# EUROPEAN PATENT SPECIFICATION

(11) **EP 3 762 004 B1**
(45) Date of publication and mention of the grant of the patent: **13.09.2023**
(21) Application number: 19710999.4
(22) Date of filing: 05.03.2019
(51) Int. Cl.: A61K 35/761, A61K 31/519, A61P 35/00

(54) **TREATMENT OF TUMORS BY A COMBINATION OF AN ONCOLYTIC ADENOVIRUS AND A CDK4/6 INHIBITOR**
BEHANDLUNG VON TUMOREN DURCH EINE KOMBINATION EINES ONKOLYTISCHEN ADENOVIRUS UND EINES CDK4/6-INHIBITORS
TRAITEMENT DE TUMEURS PAR UNE COMBINAISON D'UN ADÉNOVIRUS ONCOLYTIQUE ET D'UN INHIBITEUR DE CDK4/6

(30) Priority: 05.03.2018 EP 18000210
(43) Date of publication of application: 13.01.2021
(73) Proprietor: Klinikum rechts der Isar der Technischen Universität München, 81675 München (DE)
(72) Inventor: HOLM, Per, Sonne, 82256 Fürstenfeldbruck (DE); NAWROTH, Roman, 82239 Alling (DE)
(74) Representative: Bohmann, Armin K.
(86) International application number: PCT/EP2019/000067
(87) International publication number: WO 2019/170283

(56) References cited:
- WO-A1-2014/153204
- WO-A1-2016/178167
- WO-A2-03/099859
- WO-A2-2004/035616
- WO-A2-2005/052143
- Anonymous: "Oncolytic Viral Therapy", , 10 April 2019 (2019-04-10), XP055594785, Retrieved from the Internet: URL:https://www.pegsummit.com/Oncolytic-Vi ral-Therapy [retrieved on 2019-06-06]
- NADIIA LYPOVA ET AL: "Targeting Palbociclib-Resistant Estrogen Receptor-Positive Breast Cancer Cells via Oncolytic Virotherapy", CANCERS, vol. 11, no. 5, 16 May 2019 (2019-05-16), page 684, XP055594787, DOI: 10.3390/cancers11050684
- Koll ET AL: "Treatment of bladder cancer with YB-1 dependent oncolytic adenovirus: preliminary in vitro studies", , 1 January 2015 (2015-01-01), XP055594623, Retrieved from the Internet: URL:https://www.eusupplements.europeanurol ogy.com/article/S1569-9056(15)60035-5/pdf [retrieved on 2019-06-06]
- YUN GE ET AL: "Synergistic antitumor effects of CDK inhibitor SNS-032 and an oncolytic adenovirus co-expressing TRAIL and Smac in pancreatic cancer", MOLECULAR MEDICINE REPORTS, vol. 15, no. 6, 12 April 2017 (2017-04-12) , pages 3521-3528, XP055594561, GR ISSN: 1791-2997, DOI: 10.3892/mmr.2017.6472
- Susumu Kobayashi ET AL: "p16 INK4a Expression Adenovirus Vector to Suppress Pancreas Cancer Cell Proliferation 1", , 1 January 1999 (1999-01-01), XP055594851, Retrieved from the Internet: URL:http://clincancerres.aacrjournals.org/ content/clincanres/5/12/4182.full.pdf [retrieved on 2019-06-07]
- HAN HSI WONG ET AL: "Oncolytic Viruses for Cancer Therapy: Overcoming the Obstacles", VIRUSES, vol. 2, no. 1, 11 January 2010 (2010-01-11), pages 78-106, XP055149118, DOI: 10.3390/v2010078 & MA J ET AL: "E2F Promoter-Regulated Oncolytic Adenovirus with p16 Gene Induces Cell Apoptosis and Exerts Antitumor Effect on Gastric Cancer", DIGESTIVE DISEASES AND SCIENCES, KLUWER ACADEMIC PUBLISHERS-PLENUM PUBLISHERS, NE, vol. 54, no. 7, 26 November 2008 (2008-11-26), pages 1425-1431, XP019673573, ISSN: 1573-2568
- MING BAI ET AL: "Effects of CDKN2A (p16<sup>INK4A</sup>/p14<sup>ARF</sup>) Over-Expression on Proliferation and Migration of Human Melanoma A375 Cells", CELLULAR PHYSIOLOGY AND BIOCHEMISTRY., vol. 40, no. 6, 1 January 2016 (2016-01-01), pages 1367-1376, XP055594850, CH ISSN: 1015-8987, DOI: 10.1159/000453189
- KOSTOVA Y ET AL: "An armed, YB-1-dependent oncolytic adenovirus as a candidate for a combinatorial anti-glioma approach of virotherapy, suicide gene therapy and chemotherapeutic treatment", CANCER GENE THERAPY, APPLETON & LANGE, NEW YORK, vol. 22, no. 1, 12 December 2014 (2014-12-12), pages 30-43, XP036973442, ISSN: 0929-1903, DOI: 10.1038/CGT.2014.67 [retrieved on 2014-12-12]

## Description

The present invention is related to combination of an oncolytic virus and a CDK4/inhibitor; the combination for use in a method for the treatment of a tumor or a cancer; an adenovirus for use in a method for the treatment of a tumor or cancer in a subject together with a CDK4/6 inhibitor; and a CDK4/6 inhibitor for use in a method for the treatment of a tumor or cancer in a subject together with an adenovirus.

A number of therapeutic concepts are currently used in the treatment of tumors. Apart from using surgery, chemotherapy and radiotherapy are predominant. All these techniques are, however, associated with considerable side effects. The use of replication selective oncolytic viruses provides for a new platform for the treatment of tumors. In connection therewith a selective intratumor replication of a viral agent is initiated which results in virus replication, lysis of the infected tumor cell and spreading of the virus to adjacent tumor cells. As the replication capabilities of the virus is limited to tumor cells, normal tissue is spared from replication and thus from lysis by the virus.

Lypova N et al. (CANCERS, vol. 11, no. 5, 16 May 2019, 684) report on targeting Palbociclib-resistant estrogen receptor-positive breast cancer cells via oncolytic virotherapy.

Koll FJ et al. (URL:https://www.eusupplements.europeanurology.com/article/S1569-9056(15)60035-5/pdf; 1 January 2015) report on preliminary *in vitro* studies related to the treatment of bladder cancer with YB-1 dependent oncolytic adenovirus.

Gee Y et al. (Molecular Medicine Reports 15: 3521-3528, 2017) report on synergistic antitumor effects of CDK inhibitor SNS-032 and an oncolytic adenovirus co-expressing TRAIL and Smac in pancreatic cancer.

WO 2014/153204 A1 is related to an adenovirus comprising an E1A polypeptide comprising one or more modifications and comprising an E4orf6/7 polypeptide comprising one or more modifications.

WO 2016/178167A1 is related to an oncolytic adenovirus with functional deletions of immunodominant T-cell epitopes of adenovirus proteins and use of such adenovirus for the treatment of cancer.

WO 03/099859 A2 is related to the use of an adenovirus in the manufacture of a medicament, wherein the adenovirus is replication-deficient in cells which do not contain YB-1 in the nucleus and codes for an oncogene which transactivates an adenoviral gene selected from the group comprising E1B55kDa, E4orf6, E4orf3 and E3ADP.

WO 2004/0356616 A2 and WO 2005/052143 A2 are related to an adenovirus expressing a first protein which is selected from the group comprising an E1B protein and an E4 protein prior to an E1A protein.

Kobayashi S et al. (URL:http://clincancerres.aacrjounals.org/content/clincanres/5/12/4182. full.pdf; 1 January 1999) report on the suppression of pancreas cancer cell proliferation using a p16^{INK4a} expression adenovirus vector.

Wong HH et al. (Viruses 2010, 2, 78-106) report on overcoming obstacles in the use of oncolytic viruses for cancer therapy.

Bai M et al. (Cell Physiol Biochem 2016; 40:1367-1376) report on the effects of CDKN2A (p16^{INK4A}/p14^{ARF}) over-expression on proliferation and migration of human melanoma A375 cells.

Kostova Y et al. (Cancer Gene Therapy (2015) 22, 30-43) report on an armed, YB-1-dependent oncolytic adenovirus as a candidate for a combinatorial anti-glioma approach of virotherapy, suicide gene therapy and chemotherapeutic treatment.

The problem underlying the present invention is the provision of means so as to increase the efficacy of tumor therapy based on oncolytic viruses and adenovirus in particular.

These and other problems are solved by the subject matter of the attached independent claims; preferred embodiments may be taken from the attached dependent claims. The invention is defined in the claims.

More specifically, the problem underlying the present invention is solved in a first aspect by a combination comprising an oncolytic adenovirus and a CDK4/6 inhibitor, wherein the adenovirus is replicating in a YB-1 dependent manner and wherein the adenovirus is lacking expression of E1A13S.

In an embodiment of the first aspect, the adenovirus is replication deficient in cells which lack YB-1 in the nucleus but is replicating in cells which have YB-1 in the nucleus.

In an embodiment of the first aspect, the combination further comprises a PARP inhibitor

In an embodiment of the first aspect, the combination further comprises a bromodomain inhibitor.

More specifically, the problem underlying the present invention is solved in a second aspect by a combination according to the first aspect, including any embodiment thereof, for use in a method for the treatment of a tumor or cancer.

More specifically, the problem underlying the present invention is solved in a third aspect by an adenovirus for use in a method for the treatment of a tumor or cancer in a subject, wherein the method comprises administering to the subject an adenovirus and a CDK4/6 inhibitor, wherein the adenovirus is defined as in the first aspect, including any embodiment thereof. More specifically, the problem underlying the present invention is solved in a fourth aspect by a CDK4/6 inhibitor for use in a method for the treatment of a tumor or cancer in a subject, wherein the method comprises administering to the subject an adenovirus and the CDK4/6 inhibitor, wherein the adenovirus is defined as in the first aspect, including any embodiment thereof.

In an embodiment of the first aspect including any embodiment thereof, of the second aspect including any embodiment thereof, of the third aspect including any embodiment thereof, and of the fourth embodiment including any embodiment thereof, the adenovirus is selected from the group comprising XVir-N-31, dl520, AdΔ24, AdΔ24-RGD, dl922-947, E1Ad/01/07, dl1119/1131, CB 016, VCN-01, E1Adl1107, E1Adl1101, ORCA-010, Enadenotucirev and viruses lacking an expressed viral oncogene which is capable of binding a functional Rb tumor suppressor gene product.

In an embodiment of the first aspect including any embodiment thereof, of the second aspect including any embodiment thereof, of the third aspect including any embodiment thereof, and of the fourth embodiment including any embodiment thereof, the adenovirus is XVir-N-31.

In an embodiment of the first aspect including any embodiment thereof, of the second aspect including any embodiment thereof, of the third aspect including any embodiment thereof, and of the fourth embodiment including any embodiment thereof, wherein the CDK4/6 inhibitor is a CDK4/6 inhibitor arresting cells in the G1 phase and inhibiting E2F1.

In an embodiment of the first aspect including any embodiment thereof, of the second aspect including any embodiment thereof, of the third aspect including any embodiment thereof, and of the fourth embodiment including any embodiment thereof, the CDK4/6 inhibitor is selected from the group comprising palbociclib which is also referred to as PD 0332991, abemaciclib which is also referred to as LY-2835219, ribociclib which is also referred to as LEE011, Trilaciclib which is also referred to as G1T28, and Dinaciclib.

In an embodiment of the second aspect including any embodiment thereof, of the third aspect including any embodiment thereof, and of the fourth embodiment including any embodiment thereof, the disease tumor or cancer is expressing Rb or is Rb-positive.

In an embodiment of the second aspect including any embodiment thereof, of the third aspect including any embodiment thereof, and of the fourth embodiment including any embodiment thereof, the cells of the tumor cells have a resistance to or are insensitive to one or several pharmaceutically active agents and/or radiation.

In an embodiment of the second aspect including any embodiment thereof, of the third aspect including any embodiment thereof, and of the fourth embodiment including any embodiment thereof, the tumor or cancer contains YB-1 in the cell nucleus independent of the cell cycle.

In an embodiment of the second aspect including any embodiment thereof, of the third aspect including any embodiment thereof, and of the fourth embodiment including any embodiment thereof, the disease is selected from the group comprising bladder cancer, breast cancer, metastatic breast cancer (mBC), melanoma, glioma, pancreatic cancer, hepatocellular carcinoma, lung adenocarcinoma, sarcoma, ovarian cancer, renal cancer, prostate cancer, and leukemia.

In an embodiment of the first aspect, the adenovirus encodes an oncogene protein, wherein the oncogene protein transactivates at least one adenoviral gene, whereby the adenoviral gene is selected from the group comprising E1B55kDa, E4orf6, E4orf3 and E3ADP.

In an embodiment of the first aspect, the oncogene protein is E1A protein.

In an embodiment of the first aspect, the E1A protein is capable of binding a functional Rb tumor suppressor gene product.

In an embodiment of the first aspect, the E1A protein is incapable of binding a functional Rb tumor suppressor gene product.

In an embodiment of the first aspect, the E1A protein does not induce the localization of YB-1 into the nucleus.

In an embodiment of the first aspect, the oncogene protein exhibits one or several mutations or deletions compared to the wildtype oncogene protein E1A.

In an embodiment of the first aspect, the deletion is one selected from the group comprising deletions of the CR3 stretches and deletions of the N-terminus and deletions of the C-terminus.

In an embodiment of the first aspect, the E1A protein is capable of binding to Rb.

In an embodiment of the first aspect, the E1A protein comprises one or several mutations or deletions compared to the wildtype oncogene protein, whereby the deletion is preferably a deletion in the CR1 region and/or CR2 region.

In an embodiment of the first aspect, the E1A protein is incapable of binding to Rb.

In an embodiment of the first aspect, the virus is an adenovirus expressing E1A12 S protein.

In an embodiment of the first aspect, the virus is an adenovirus lacking a functionally active adenoviral E3 region.

In an embodiment of the first aspect, the virus is an adenovirus lacking expression of E1B 19 kDa protein.

In an embodiment of the first aspect, the virus is an adenovirus expressing an RGD motif at a fibre.

In an embodiment of the first aspect, the virus is an adenovirus serotype 5.

In an embodiment of the first aspect, the adenovirus is dl520, wherein the adenoviral E3 region is functionally inactive.

In an embodiment of the first aspect, the adenovirus is dl520, wherein dl520 is lacking expression of E1B 19 kDa protein.

In an embodiment of the first aspect, the adenovirus is dl520 expressing an RGD motif at a fibre.

In an embodiment of the first aspect, the virus encodes YB-1.

In an embodiment of the first aspect, the gene coding for YB-1 is under the control of a tissue-specific promoter, tumor-specific promoter and/or a YB-1 dependent promoter.

In an embodiment of the first aspect, the YB-1 dependent promoter is the adenoviral E2 late promoter.

In an embodiment of the first aspect, the CDK4/6 inhibitor is a compound which reduces Rb phosphorylation in a cell, preferably a tumor cell.

In an embodiment of the first aspect, the CDK4/6 inhibitor is a compound which reduces Rb expression in a cell, preferably a tumor cell.

In an embodiment of the first aspect, the CDK4/6 inhibitor causes G1 arrest in a cell and inhibits E2F1.

In an embodiment of the first aspect, the composition further comprises a PARP inhibitor.

In an embodiment of the first aspect, the PARP inhibitor is selected from the group comprising olaparib, veliparib, rucaparib and BMN673.

In an embodiment of the first aspect, the composition further comprises a bromodomain inhibitor.

In an embodiment of the first aspect, the bromodomain inhibitor is selected from the group comprising JQ1, OTX-015, I-BET151, CPI-0610, I-BET762, CPI203, PFI-1 and MS 436.

In an embodiment of the first aspect, the constituents of the combination are for separate administration.

In an embodiment of the second aspect, the adenovirus is replicating in a YB-1 dependent manner.

In an embodiment of the second aspect, the adenovirus is replication deficient in cells which lack YB-1 in the nucleus, but is replicating in cells which have YB-1 in the nucleus.

In an embodiment of the second aspect, the adenovirus encodes an oncogene protein, wherein the oncogene protein transactivates at least one adenoviral gene, whereby the adenoviral gene is selected from the group comprising E1B55kDa, E4orf6, E4orf3 and E3ADP.

In an embodiment of the second aspect, the oncogene protein is E1A protein.

In an embodiment of the second aspect, the E1A protein is capable of binding a functional Rb tumor suppressor gene product.

In an embodiment of the second aspect, the E1A protein is incapable of binding a functional Rb tumor suppressor gene product.

In an embodiment of the second aspect, the E1A protein does not induce the localization of YB-1 into the nucleus.

In an embodiment of the second aspect, the oncogene protein exhibits one or several mutations or deletions compared to the wildtype oncogene protein E1A.

In an embodiment of the second aspect, the deletion is one selected from the group comprising deletions of the CR3 stretches and deletions of the N-terminus and deletions of the C-terminus.

In an embodiment of the second aspect, the E1A protein is capable of binding to Rb.

In an embodiment of the second aspect, the E1A protein comprises one or several mutations or deletions compared to the wildtype oncogene protein, whereby the deletion is preferably a deletion in the CR1 region and/or CR2 region.

In an embodiment of the second aspect, the E1A protein is incapable of binding to Rb.

In an embodiment of the second aspect, the virus is an adenovirus expressing E1A12 S protein.

In an embodiment of the second aspect, the virus is an adenovirus lacking a functionally active adenoviral E3 region.

In an embodiment of the second aspect, the virus is an adenovirus lacking expression of E1B 19 kDa protein.

In an embodiment of the second aspect, the virus is an adenovirus expressing an RGD motif at a fibre.

In an embodiment of the second aspect, the virus is an adenovirus serotype 5.

In an embodiment of the second aspect, the adenovirus is dl520, wherein the adenoviral E3 region is functionally inactive.

In an embodiment of the second aspect, the adenovirus is dl520, wherein dl520 is lacking expression of E1B 19 kDa protein.

In an embodiment of the second aspect, the adenovirus is dl520 expressing an RGD motif at a fibre.

In an embodiment of the second aspect, the virus encodes YB-1.

In an embodiment of the second aspect, the gene coding for YB-1 is under the control of a tissue-specific promoter, tumor-specific promoter and/or a YB-1 dependent promoter.

In an embodiment of the second aspect, the YB-1 dependent promoter is the adenoviral E2 late promoter.

In an embodiment of the second aspect, the CDK4/6 inhibitor is a compound which reduces Rb phosphorylation in a cell, preferably a tumor cell.

In an embodiment of the second aspect, the CDK4/6 inhibitor is a compound which reduces Rb expression in a cell, preferably a tumor cell.

In an embodiment of the second aspect, the composition further comprises a PARP inhibitor.

In an embodiment of the second aspect, the PARP inhibitor is selected from the group comprising olaparib, veliparib, rucaparib and BMN673.

In an embodiment of the second aspect, the composition further comprises a bromodomain inhibitor.

In an embodiment of the second aspect, the bromodomain inhibitor is selected from the group comprising JQ1, OTX-015, I-BET151, CPI-0610, I-BET762, CPI203, PFI-1 and MS 436.

In an embodiment of the second aspect, the constituents of the combination are for separate administration.

In an embodiment of the second aspect, cells of the tumor have a disruption of the CDK4/6 signaling pathway.

In an embodiment of the second aspect, cells of the tumor have an uncontrolled G1-S transition of the cell cycle.

In an embodiment of the second aspect, cells of the tumor have a loss of function mutation or a deletion in a gene selected from the group comprising RB1 gene, CDKN2A gene and CDKN2B gene.

In an embodiment of the second aspect, cells of the tumor have an amplification of a gene and/or an activating mutation in a gene.

In an embodiment of the second aspect, the gene is selected from the group comprising CCND1, E2F1, E2F2, E2F3, CDK4 and CDK6.

In an embodiment of the second aspect, the gene is one coding for a component of a mitogenic signaling pathway.

In an embodiment of the second aspect, the mitogenic signaling pathway is selected from the group comprising the PI3K pathway and the MAPK pathway.

In an embodiment of the second aspect, the cells of the tumor cells have a resistance to or are insensitive to one or several pharmaceutically active agents and/or radiation.

In an embodiment of the second aspect, the pharmaceutically active agent is a cytostatic.

In an embodiment of the second aspect, the resistance is mediated by an ABC transporter.

In an embodiment of the second aspect, the ABC transporter is selected from the group comprising MRP and MDR, in particular MDR-1.

In an embodiment of the second aspect, the resistance is a multiple resistance or polyresistance, particular a multiple or polyresistance against a cytostatic and/or radiation.

In an embodiment of the second aspect, the cells of the tumor are Rb-positive.

In an embodiment of the second aspect, the cells of the tumor have YB-1 in the nucleus.

In an embodiment of the second aspect, the cells of the tumor have YB-1 in the nucleus after induction.

In an embodiment of the second aspect, the transport of YB-1 into the nucleus is triggered by at least one measure selected from the group comprising irradiation, administration of cytostatics and hyperthermia.

In an embodiment of the second aspect, the measure is applied to a cell, an organ or an organism, preferably an organism in need thereof, more preferably an organism suffering from the tumor.

In an embodiment of the third aspect, the adenovirus encodes an oncogene protein, wherein the oncogene protein transactivates at least one adenoviral gene, whereby the adenoviral gene is selected from the group comprising E1B55kDa, E4orf6, E4orf3 and E3ADP.

In an embodiment of the third aspect, the oncogene protein is E1A protein.

In an embodiment of the third aspect, the E1A protein is capable of binding a functional Rb tumor suppressor gene product.

In an embodiment of the third aspect, the E1A protein is incapable of binding a functional Rb tumor suppressor gene product.

In an embodiment of the third aspect, the E1A protein does not induce the localization of YB-1 into the nucleus.

In an embodiment of the third aspect, the oncogene protein exhibits one or several mutations or deletions compared to the wildtype oncogene protein E1A.

In an embodiment of the third aspect, the deletion is one selected from the group comprising deletions of the CR3 stretches and deletions of the N-terminus and deletions of the C-terminus.

In an embodiment of the third aspect, the E1A protein is capable of binding to Rb.

In an embodiment of the third aspect, the E1A protein comprises one or several mutations or deletions compared to the wildtype oncogene protein, whereby the deletion is preferably a deletion in the CR1 region and/or CR2 region.

In an embodiment of the third aspect, the E1A protein is incapable of binding to Rb.

In an embodiment of the third aspect, the virus is an adenovirus expressing E1A12 S protein.

In an embodiment of the third aspect, the virus is an adenovirus lacking a functionally active adenoviral E3 region.

In an embodiment of the third aspect, the virus is an adenovirus lacking expression of E1B 19 kDa protein.

In an embodiment of the third aspect, the virus is an adenovirus expressing an RGD motif at a fibre.

In an embodiment of the third aspect, the virus is an adenovirus serotype 5.

In an embodiment of the third aspect, the adenovirus is dl520, wherein the adenoviral E3 region is functionally inactive.

In an embodiment of the third aspect, the adenovirus is dl520, wherein dl520 is lacking expression of E1B 19 kDa protein.

In an embodiment of the third aspect, the adenovirus is dl520 expressing an RGD motif at a fibre.

In an embodiment of the third aspect, the virus encodes YB-1.

In an embodiment of the third aspect, the gene coding for YB-1 is under the control of a tissue-specific promoter, tumor-specific promoter and/or a YB-1 dependent promoter.

In an embodiment of the third aspect, the YB-1 dependent promoter is the adenoviral E2 late promoter.

In an embodiment of the third aspect, the CDK4/6 inhibitor is a compound which reduces Rb phosphorylation in a cell, preferably a tumor cell.

In an embodiment of the third aspect, the CDK4/6 inhibitor is a compound which reduces Rb expression in a cell, preferably a tumor cell.

In an embodiment of the third aspect, the method further comprises administering a PARP inhibitor to the subject.

In an embodiment of the third aspect, the PARP inhibitor is selected from the group comprising olaparib, veliparib, rucaparib and BMN673.

In an embodiment of the third aspect, the method further comprises administering a bromodomain inhibitor to the subject.

In an embodiment of the third aspect, the bromodomain inhibitor is selected from the group comprising JQ1, OTX-015, I-BET151, CPI-0610, I-BET762, CPI203, PFI-1 and MS 436.

In an embodiment of the third aspect, the adenovirus, the CDK4/6 inhibitor, the PARP inhibitor and/or the bromodomain inhibitor are administered to the subject separately or as any combination.

In an embodiment of the third aspect, cells of the tumor have a disruption of the CDK4/6 signaling pathway.

In an embodiment of the third aspect, cells of the tumor have an uncontrolled G1-S transition of the cell cycle.

In an embodiment of the third aspect, cells of the tumor have a loss of function mutation or a deletion in a gene selected from the group comprising RB1 gene, CDKN2A gene and CDKN2B gene.

In an embodiment of the third aspect, cells of the tumor have an amplification of a gene and/or an activating mutation in a gene.

In an embodiment of the third aspect, the gene is selected from the group comprising CCND1, E2F1, E2F2, E2F3, CDK4 and CDK6.

In an embodiment of the third aspect, the gene is one coding for a component of a mitogenic signaling pathway.

In an embodiment of the third aspect, the mitogenic signaling pathway is selected from the group comprising the PI3K pathway and the MAPK pathway.

In an embodiment of the third aspect, the cells of the tumor cells have a resistance to or are insensitive to one or several pharmaceutically active agents and/or radiation.

In an embodiment of the third aspect, the pharmaceutically active agent is a cytostatic.

In an embodiment of the third aspect, the resistance is mediated by an ABC transporter.

In an embodiment of the third aspect, the ABC transporter is selected from the group comprising MRP and MDR, in particular MDR-1.

In an embodiment of the third aspect, the resistance is a multiple resistance or polyresistance, particular a multiple or polyresistance against a cytostatic and/or radiation.

In an embodiment of the third aspect, the cells of the tumor are Rb-positive.

In an embodiment of the third aspect, the cells of the tumor have YB-1 in the nucleus.

In an embodiment of the third aspect, the cells of the tumor have YB-1 in the nucleus after induction.

In an embodiment of the third aspect, the transport of YB-1 into the nucleus is triggered by at least one measure selected from the group comprising irradiation, administration of cytostatics and hyperthermia.

In an embodiment of the third aspect, the measure is applied to a cell, an organ or an organism, preferably an organism in need thereof, more preferably an organism suffering from the tumor.

In an embodiment of the fourth aspect, the adenovirus encodes an oncogene protein, wherein the oncogene protein transactivates at least one adenoviral gene, whereby the adenoviral gene is selected from the group comprising E1B55kDa, E4orf6, E4orf3 and E3ADP.

In an embodiment of the fourth aspect, the oncogene protein is E1A protein.

In an embodiment of the fourth aspect, the E1A protein is capable of binding a functional Rb tumor suppressor gene product.

In an embodiment of the fourth aspect, the E1A protein is incapable of binding a functional Rb tumor suppressor gene product.

In an embodiment of the fourth aspect, the E1A protein does not induce the localization of YB-1 into the nucleus.

In an embodiment of the fourth aspect, the oncogene protein exhibits one or several mutations or deletions compared to the wildtype oncogene protein E1A.

In an embodiment of the fourth aspect, the deletion is one selected from the group comprising deletions of the CR3 stretches and deletions of the N-terminus and deletions of the C-terminus.

In an embodiment of the fourth aspect, the E1A protein is capable of binding to Rb.

In an embodiment of the fourth aspect, the E1A protein comprises on In an embodiment of the fourth aspect, the deletion is preferably a deletion in the CR1 region and/or CR2 region.

In an embodiment of the fourth aspect, the E1A protein is incapable of binding to Rb.

In an embodiment of the fourth aspect, the virus is an adenovirus expressing E1A12 S protein.

In an embodiment of the fourth aspect, the virus is an adenovirus lacking a functionally active adenoviral E3 region.

In an embodiment of the fourth aspect, the virus is an adenovirus lacking expression of E1B 19 kDa protein.

In an embodiment of the fourth aspect, the virus is an adenovirus expressing an RGD motif at a fibre.

In an embodiment of the fourth aspect, the virus is an adenovirus serotype 5.

In an embodiment of the fourth aspect, the adenovirus is dl520, wherein the adenoviral E3 region is functionally inactive.

In an embodiment of the fourth aspect, the adenovirus is dl520, wherein dl520 is lacking expression of E1B 19 kDa protein.

In an embodiment of the fourth aspect, the adenovirus is dl520 expressing an RGD motif at a fibre.

In an embodiment of the fourth aspect, the virus encodes YB-1.

In an embodiment of the fourth aspect,the gene coding for YB-1 is under the control of a tissue-specific promoter, tumor-specific promoter and/or a YB-1 dependent promoter.

In an embodiment of the fourth aspect,the YB-1 dependent promoter is the adenoviral E2 late promoter.

In an embodiment of the fourth aspect, the CDK4/6 inhibitor is a compound which reduces Rb phosphorylation in a cell, preferably a tumor cell.

In an embodiment of the fourth aspect, the CDK4/6 inhibitor is a compound which reduces Rb expression in a cell, preferably a tumor cell.

In an embodiment of the fourth aspect, the method further comprises administering a PARP inhibitor to the subject.

In an embodiment of the fourth aspect, the PARP inhibitor is selected from the group comprising olaparib, veliparib, rucaparib and BMN673.

In an embodiment of the fourth aspect, the method further comprises administering a bromodomain inhibitor to the subject.

In an embodiment of the fourth aspect, the bromodomain inhibitor is selected from the group comprising JQ1, OTX-015, I-BET151, CPI-0610, I-BET762, CPI203, PFI-1 and MS 436.

In an embodiment of the fourth aspect, the adenovirus, the CDK4/6 inhibitor, the PARP inhibitor and/or the bromodomain inhibitor are administered to the subject separately or as any combination.

In an embodiment of the fourth aspect, cells of the tumor have a disruption of the CDK4/6 signaling pathway.

In an embodiment of the fourth aspect, cells of the tumor have an uncontrolled G1-S transition of the cell cycle.

In an embodiment of the fourth aspect, cells of the tumor have a loss of function mutation or a deletion in a gene selected from the group comprising RB1 gene, CDKN2A gene and CDKN2B gene.

In an embodiment of the fourth aspect, cells of the tumor have an amplification of a gene and/or an activating mutation in a gene.

In an embodiment of the fourth aspect, the gene is selected from the group comprising CCND1, E2F1, E2F2, E2F3, CDK4 and CDK6.

In an embodiment of the fourth aspect, the gene is one coding for a component of a mitogenic signaling pathway.

In an embodiment of the fourth aspect, the mitogenic signaling pathway is selected from the group comprising the PI3K pathway and the MAPK pathway.

In an embodiment of the fourth aspect, the cells of the tumor cells have a resistance to or are insensitive to one or several pharmaceutically active agents and/or radiation.

In an embodiment of the fourth aspect, the pharmaceutically active agent is a cytostatic.

In an embodiment of the fourth aspect, the resistance is mediated by an ABC transporter.

In an embodiment of the fourth aspect, the ABC transporter is selected from the group comprising MRP and MDR, in particular MDR-1.

In an embodiment of the fourth aspect, the resistance is a multiple resistance or polyresistance, particular a multiple or polyresistance against a cytostatic and/or radiation.

In an embodiment of the fourth aspect, the cells of the tumor are Rb-positive.

In an embodiment of the fourth aspect, the cells of the tumor have YB-1 in the nucleus.

In an embodiment of the fourth aspect, the cells of the tumor have YB-1 in the nucleus after induction.

In an embodiment of the fourth aspect, the transport of YB-1 into the nucleus is triggered by at least one measure selected from the group comprising irradiation, administration of cytostatics and hyperthermia.

In an embodiment of the fourth aspect, the measure is applied to a cell, an organ or an organism, preferably an organism in need thereof, more preferably an organism suffering from the tumor.

It will be acknowledged by a person skilled in the art that each and any embodiment of one aspect of the present invention is also an embodiment of each and any of the other aspects of the present invention, including any embodiment thereof.

Without wishing to be bound by any theory, the present inventors have surprisingly found that combining an oncolytic virus, preferably an oncolytic adenovirus, with a CDK4/6 inhibitor increases the efficacy of tumor therapy based on such oncolytic adenovirus. More specifically, the CDK4/6 inhibitor is assumed to inhibit E2F-1 thus reducing its effective concentration, preferably in tumor cells, and synchronizes G1 arrest in cells. Because of this, more infected cells can complete the entire viral life cycle.

Based on the evidence and insights provided herein, a person skilled in the art will understand that any - mutant - adenovirus is suitable for use in the practicing of the instant invention which allows that at least as little as 10 %, 20 % or 30 % of wild type expression and, respectively, activity of E1B55K and E4orf6 is achieved by such adenovirus. It will be appreciated by a person skilled in the art that such mutant adenovirus can be generated by modifying E1A. Exemplary mutant adenoviruses are adenovirus XVir-N-31, dl520, AdΔ24, AdΔ24-RGD, dl922-947, E1Ad/01/07, dl1119/1131, CB 016, VCN-01, E1Adl1107, E1Adl1101, ORCA-010, Enadenotucirev and viruses lacking an expressed viral oncogene which is capable of binding a functional Rb tumor suppressor gene product.

Eponymous for adenoviruses is the first isolation of the virus in human tonsils and adenoid tissue in 1953 by Wallace P. Rowe and Robert J. Huebner (Rowe et al., 1953). The family of Adenoviridae comprises five genera, namely Mastadenoviruses, Aviadenoviruses, Siadenoviruses, Atadenoviruses and Ichtadenoviruses (Modrow, 2013). Due to their oncogenicity in newborn rodents, they can be classified into seven subgroups HAdV-A to HAdV-G (Boulanger and Blair, 1991) with altogether 62 serotypes. Thereby, research on oncolytic virotherapy is mainly focusing on Mastadenovirus Type C serotype 5.

The uncoated icosahedral capsid with a size of 80 to 110 nm is comprised of 252 capsomers, that consist of 12 pentons, assembled of a penton basis and spike-like protein structures, called fibers, on the vertices of the capsid and 249 faces, called hexons (Modrow, 2013). The whole lifecycle of adenoviruses, can be subdivided into an early phase with cell entry, nuclear translocation of the viral genome, transcription and translation of early genes and the late phase with transcription and translation of late genes. Late proteins are thereby mainly responsible for assembly of structural proteins and maturation of virions (Russell, 2000). In permissive cells, the early phase takes about 6-8 hours with a following late phase of about 4-6 hours. Attachment occurs via interaction of a knob structure, that is present on every end of the fiber structures with a receptor on the target cells at least for the adenoviruses HAdV-A, - C, -E and -F. Since this receptor was detected as the same one, that is responsible for coxsackie B virus adsorption, the receptor is called coxsackievirus and adenovirus receptor (CAR) (Bergelson, 1997). Additionally, binding on the surface of the target cell is supported by "bridge molecules", soluble proteins in bodily fluids like blood coagulation factors VII and X, that mediates the binding of the fiber proteins of certain adenovirus types (Modrow, 2013). After this adsorption step, an RGD-motif (arginine-glycine-aspartic acid) in the penton base interacts with heterodimeric integrins αvβ3 or αvβ5, that function as co-receptors in this process. This interaction results in internalization of the virus (Wickham et al., 1993). Subsequently, endocytosis via clathrin-mediated internalization in the cytoplasmic membrane occurs and the virus is present in endosomes. After acidification of the endocytic vesicles, the viral fiber protein changes its conformation with resulting destruction of the endosomal membrane (Greber et al., 1996). Viral particles are now free in the cytoplasm. Via binding of residual particles on dyneins of microtubules, the viral genome is transferred into the nucleus (Modrow, 2013).

The genome of adenoviruses consists of a double-stranded, linear DNA of 36-38 kb length. By interaction of two terminal protein (TP) molecules, that are covalently linked to both 5' ends, a quasi-circular state is formed (Modrow, 2013). In general, five coding regions of the adenoviral genome can be subdivided into the early genes E1-E4, active mainly in the early phase of infection and the late genes (L1-L5), that encode proteins mainly necessary for viral particle formation (Modrow, 2013).

Adenoviral replication is especially dependent on the expression of the early viral gene E2, which is strongly induced by the large E1A protein (E1A13S). The first viral gene post infection to be transcribed is early region 1A (E1A). The primary E1A transcript is processed by differential splicing to yield five distinct messages with sedimentation coefficients of 13S, 12S 11S, 10S, and 9S. The 13S and 12S mRNAs are the most abundant at early times during infection, while the 9S mRNA is the most abundant at late times. The 11S and 10S mRNA are minor species that become more abundant at late times after infection. The 13S, 12S, 115, 10S and 9S E1A mRNA code for 289 residue (R), 243R, 217R, 171R and 55R proteins respectively, all of which are detectable in vivo with the exception of the 9S product which has only been detected in vitro. In general, adenoviral gene expression is highly regulated in course of infection with a high degree of complexity. Thereby, transcription of the E2 genes which products encode for the viral DNA polymerase and other proteins necessary for efficient viral replication is under control of two promoters, the E2-early and E2-late promoter.

Due to its two overlapping transcriptional control regions, the E2-early promoter can be subdivided into the major promoter starting at position +1 and the minor promoter starting at position -26, both containing a TATA motif (Swaminathan and Thimmapaya, 1996). These motifs serve as binding sites for TATA box-binding proteins (TBP). Moreover, one binding site for the activating transcription factor (ATF) between positions -68 and -77 and two E2F/DP-1 binding sites (TTTCGCGC) **[AKB: Less than 10 nt, no need for incorporating the sequence into a sequence alignment.],** aligned in inverted orientation with respect to each other, are located at position -35 and -63 of the major E2-early promoter (Swaminathan and Thimmapaya, 1996). The activation of the E2-early promoter through E1A is mainly dependent on the two E2F-binding sites localized in the major promoter part.

At intermediate stages of infection, after about 6 hpi (hours past infection), expression of E2 genes is controlled by the E2-late promoter. At position nt -33 to -22 of its 157-bp sequence, there is a TATA box, that can be bound and activated by cellular TBP (Swaminathan and Thimmapaya, 1996). Moreover, two SP1 recognition sites and three CCAAT boxes are characteristic for the E2-late promoter.

Since it was shown, that the cellular factor YB-1 is able to bind to inverted CCAAT boxes, interaction between the Y-box binding protein 1 (YB-1) and the E2-late promoter was investigated. Holm et al. showed in 2002, that there is in fact a specific interaction of YB-1 with the Y-boxes (inverted CCAAT-boxes), present in the E2-late promoter with ability to control the activity of this promoter (Holm et al., 2002). To exert its transactivating activity, YB-1 has to be translocated into the nucleus via the adenoviral complex E1B-55k/E4-orf6. These early viral genes are expressed after transactivation of E1A-13S (Frisch and Mymryk, 2002).

The cellular factor YB-1, encoded by the YBX1 gene, is a cold shock domain bearing DNA-binding protein with multiple functions in transcription, splicing, translational control and repair of DNA damages (Kohno et al., 2003). Moreover, it plays an important role in drug-resistance, due to its activation of MDR1 and MRP1 genes that are involved in the development of a multidrug-resistant phenotype in cancer cells (Mantwill et al., 2006). YB-1 expression is induced with subsequent nuclear transport through exposure of extrinsic stress factors like adenoviral infection, chemotherapy or UV radiation (Mantwill et al., 2006).

Transcriptional activation of adenovirus early genes and late genes is pivotal to the viral life cycle. Briefly, the viral life cycle is initiated by the activation of E1A transcription, followed by a cascade of activation of E2, E3 and E4 genes. Finally, the major late promoter (MLP) is activated to coordinate the expression of capsid and accessory proteins involved largely in genome encapsidation (Turner et al. 2015). To overcome the block to viral DNA replication present in non-proliferating cells, the virus expresses the early 1A proteins (E1A). These immediate early proteins drive cells into S-phase and induce expression of all other viral early genes. During infection, several E1A isoforms are expressed with proteins of 289, 243, 217, 171, and 55 residues being present for human adenovirus type 5. In the context of infection, the primary driver of viral gene expression is the large E1A 289R protein (Radko et al. 2015).

Upon infection, expression of the adenoviral E1A protein promotes cell cycle progression from G0/G1 phase into S-Phase and viral replication even in terminally differentiated epithelial cells, the major target of human adenoviruses. This process is considered to be essential for adenoviral life cycle.

Adenoviruses have been designed to infect, replicate and kill cancer cells while sparing normal cells. Following infection and replication in tumor cells, oncolytic viruses kill the cells, releasing virions for subsequent cycles of amplification. To achieve replication only into tumor cells, two kinds of genetic modifications have been made, leading to three subclasses of oncolytic adenovirus (also referred to as CRAd herein) have been designed all of which may be used in the practicing of the present invention. Furthermore, oncolytic adenoviruses suitable for use in the practicing of the present invention are, among others, described in WO 2003/099859.

Type I CRAd are characterized by mutations or deletions in the E1 region of the genome, interfering with the inactivation of cell cycle regulators such as p53 and retinoblastoma protein (Rb). As a consequence, type I CRAds replicate in actively dividing tumor cells. For example, Onyx-015, also known as dl1520, which is unable to express the E1B-55 kDa protein, is unable to inactivate p53 and avoid p53-induced cell cycle arrest. Several studies attributed the molecular basis of Onyx-015 selectivity to the lack of expression of p53 or one of genes involved in p53 pathway. However, O'Shea et al. showed that late viral RNA export, rather than p53 inactivation, determines Onyx-015 virus selectivity. Other type I CRAds with deletions in the E1A region are unable to bind Rb and trigger S phase entry. For example, dl922-947 and Δ24 contain a 24 nucleotide deletion in CR2 domain of E1A region, abrogating E1A-Rb interaction. As a result, these viruses replicate mainly in tumor cells where free, unbound E2F is available.

Another way to restrict adenoviral replication to tumor cells is to regulate the transcription of viral genes required for viral replication. In type II CRAds, the genome is placed under the control of a tumor-specific promoter. Those promoters were derived from genes known to be preferentially expressed in some tumors compared to normal tissue (e.g., telomerase or cyclooxygenase II); or that are overexpressed in tumors (e.g., prostate specific antigen, PSA or α-foetoprotein, AFP) compared to normal tissues. In type III CRAds such as XVir-N-31 (Ad-Delo3-RGD) is characterized by deletion of the transactivation domain CR3 in the E1A13S protein. XVir-N-31 is replication defective adenoviruses in normal cells. XVir-N-31 restores its replication competence by the presence of the cellular multifunctional protein YB-1 in the nucleus. Accordingly, CRAds are only capable to replicate in tumor cells and thus ultimately lysing them. Neither mutations of p53, nor ras nor RB are effective to complement the replication deficiency of XVir-N-31. XVir-N-31 lack E1A13S, consequently the E1B55k protein and the E4orf6 protein are not expressed. This deficiency is complemented by the presence of YB-1 in the nucleus of tumor cells which triggers the expression of E1B55k and E4orf6 independently of E1A13S. Once induced by the presence of YB-1 in the nucleus, E1B55k and E4orf6 transfer further cellular YB-1 into the nucleus propelling viral replication.

The cell cycle progresses sequentially through the gap 1 (G1), synthesis (S), gap 2 (G2) and mitosis (M) stages. This progression is regulated via a complex signaling network. The CDK (cyclin-dependent kinase) proteins, CDK1, CDK2, CDK4 and CDK6, are major regulators of cell cycle progression when complexed with specific cyclin proteins. Constitutive expression of CDKs and temporal control of various cyclins enables the regulation of specific cell cycle phases by distinct cyclin-CDK complexes. CDK activity is negatively regulated by several inhibitory proteins. The various aspects of CDK biology and function have been previously reviewed comprehensively.

CDK4 and CDK6, which show structural and functional homology, regulate the transition of quiescent cells in the G1 phase into the S phase when complexed with cyclin D proteins. Cyclin D proteins have three subtypes, cyclin D1-3, and accumulate in the presence of mitogenic stimuli. Negative regulators of CDK4/6 include the inhibitor of CDK4 (INK4) proteins, p16INK4A, p15INK4B, p18INK4C and p19INK4D, which inhibit CDK4/6 activity either by reducing their binding with cyclin D1 or by directly occupying their catalytic domains.

The kinase activity of CDK4/6 leads to the phosphorylation of members of the retinoblastoma (Rb) protein family including Rb, p107 and p130, which results in their functional inactivation. In quiescent cells, active hypophosphorylated Rb binds to members of the E2F transcription factor family that form a complex with DP-1/2, together with other co-repressors and suppresses E2F function (Rubin et al. 2005). Upon phosphorylation, Rb dissociates from this complex and allows the transcription of E2F target genes including cyclin A, cyclin E and DHFR, among others, which are required for the transition of the cell cycle into the S phase. Hence, inhibition of CDK4/6 activity leads to Rb dephosphorylation and repression of E2F activity, which promotes a G0/G1 arrest. This has fueled the development of CDK4/6 inhibitors as target therapy in cancer cells.

The disruption of the CDK4/6-Rb signalling pathway and an uncontrolled G1-S transition of the cell cycle is a common feature of cancer cells. This can occur due to various molecular alterations including loss of function mutations or deletions of the RB1 gene (encoding for Rb), CDKN2A (encoding for p16INK4A and p14ARF) or CDKN2B (encoding for p15INK4B). Such deregulation can also result from amplification or activating mutations in CCND1 (encoding for cyclin D1), E2F1-3, CDK4, CDK6 or components of various mitogenic signalling pathways such as the PI3K or MAPK pathways.

Several ATP-competitive small molecule CDK inhibitors have been developed. However, first generation inhibitors such as flavopiridol are non-selective and can inhibit multiple CDKs which might result in limited efficacy and high toxicity. Next generation CDK4/6 inhibitors display high selectivity and include palbociclib (PD-0332991 from Pfizer), abemaciclib (LY-2835219 from Eli Lilly) and ribociclib (LEE011 from Novartis) and Trilaciclib (G1T28). These CDK4/6 inhibitors have been tested pre-clinically in in vitro and in vivo models of several cancer entities including leukemia, breast cancer, melanoma, glioma, pancreatic cancer, hepatocellular carcinoma, lung adenocarcinoma, sarcoma, ovarian cancer, renal cancer, prostate cancer and metastatic breast cancer (mBC). In most studies they have demonstrated a consistent molecular and functional phenotype with a dose-dependent reduction in Rb phosphorylation, protein expression and transcription of E2F target genes, which correlates with a G0/G1 arrest and inhibition of cell proliferation. Additionally, all these reports demonstrate that Rb expression is a pre-requisite for sensitivity to these inhibitors.

CDK4/6 inhibitors such as PD-0332991, result in a dose dependent reduction in total Rb protein that correlated with a decrease in phosphorylated Rb. This decrease in total Rb correlates partially with a reduction in RB1 transcript levels and transcription of E2F target genes CCNA2 and CCNE2. Also, E2F expression level is significantly downregulated.

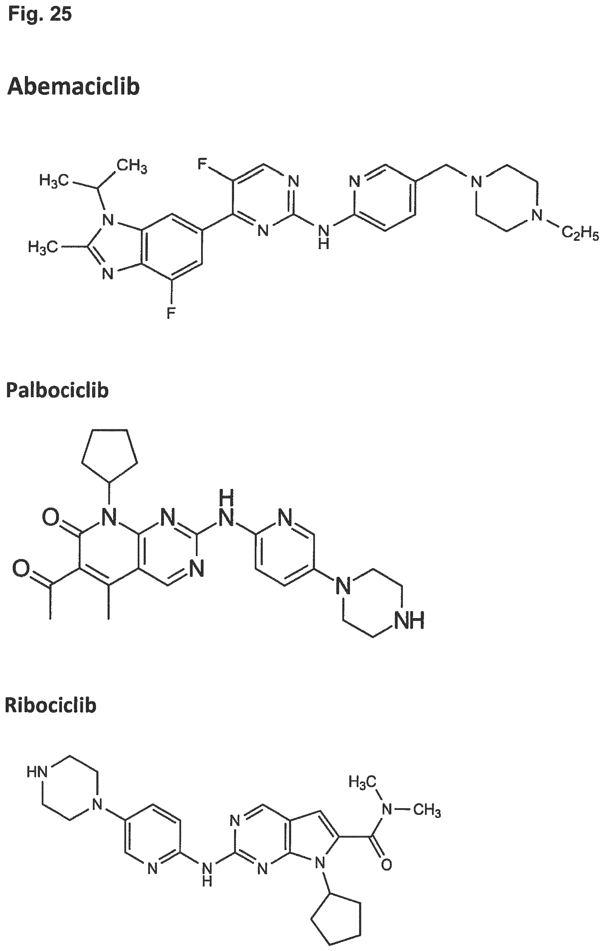

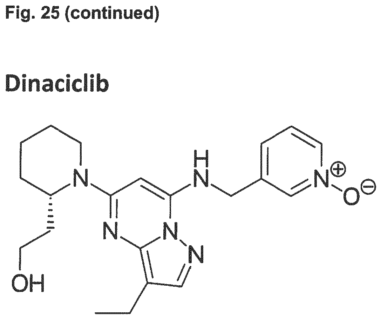

CDK4/6 inhibitors suitable for use in the practicing of the present invention are disclosed in Fig. 25.

As evident from the example part, any CDK4/6 inhibitor is suitable for use in combination with a virus, preferably an adenovirus and more preferably an oncolytic adenovirus, whereby the CDK4/6 inhibitor causes G1 arrest of cells and inhibits E2F1, more specifically, E2F1 activity.

It will be appreciated by a person skilled in the art that any CDK4/6 inhibitor is used in a therapeutically effective concentration.

PARP1 is a protein that is important for repairing single-strand breaks ('nicks' in the DNA). In mammals, 17 PARP family members have been discovered, and only 6 of these synthesize poly ADP-ribose (pADPr). PARP1, PARP2, and PARP3 have roles in DNA repair. PARP1 binds to DNA that has suffered from single-stranded breaks (SSBs) and double-stranded breaks (DSBs). PARP1 then undergoes a conformational change that aligns key amino acid residues in the active site, thereby increasing its activity. Once PARP1 is activated, it synthesizes pADPr, which binds to proteins and alters their function. pADPr is rapidly degraded by pADPr glycohydrolase to ensure that the levels of the pADPr present are reflective of DNA damage and that the response to pADPr is terminated following DNA repair.

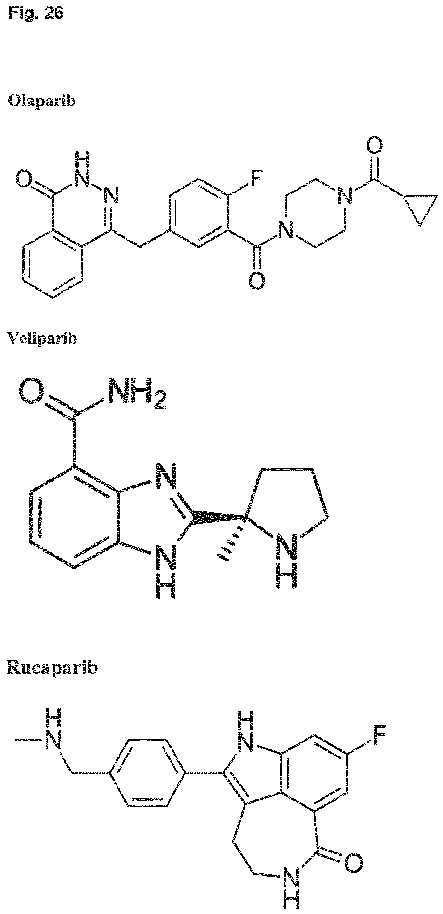

By inhibiting DNA repair pathways, PARP1 inhibitors cause an increase in single-stranded breaks within DNA. This DNA damage is unrepaired and carried into daughter cells following replication, as BER is no longer occurring. This leads to an increase in DSBs in tumors that have BRCA1 and BRCA2 mutations (Scott et al. 2015, J Clin Oncol., 33(12): 1397-140). The chemical structures of PARP inhibitors including the PARP drug candidates rucaparib, veliparib and olaparib are shown in Fig. 26 and described in Antolin and Mestres 2014, Oncotarget, 30;5(10):3023-8, including the benzamide moiety that characterizes all PARP inhibitor structures.

In addition, it is well established that YB-1 potentiates PARP activity and decreases the efficacy of PARP1 inhibitors (Alemasova et al. 2018, Oncotarget, 34, 23349-65), suggesting that YB-1 dependent oncolytic adenovirus in combination with both CDK 4/6 Inhibitors and PARP-Inhibitors will work synergistically in cancer cell killing. Olaparib and BMN673 (Talazolarib developed from Pfizer, USA, Clin Cancer Res. 2013, 15;19(18):5003-15) are example of PARP-Inhibitors.

It will be appreciated by a person skilled in the art that any PARP inhibitor is used in a therapeutically effective concentration.

CDK4/6 inhibitors suitable for use in the practicing of the present invention are disclosed in Fig. 25.

Aberrations in the epigenetic landscape are a hallmark of cancer and acetylation of lysine residues is a post-translational modification with broad relevance to cellular signaling and disease biology. Enzymes that 'write' (histone acetyltransferases, HATs) and 'erase' (histone deacetylases, HDACs) acetylation sites are an area of extensive research in current drug development. Recruitment of proteins to macromolecular complexes by acetylated lysine residues is mediated by bromodomains (BRDs), which are evolutionarily highly conserved protein-interaction modules that recognize ε-N-lysine acetylation motifs. The conserved BRD fold contains a deep, largely hydrophobic acetyl lysine binding site, which represents an attractive pocket for the development of small, pharmaceutically active molecules. Proteins that contain BRDs have been implicated in the development of a large variety of diseases.

Recently, two highly potent and selective inhibitors that target BRDs of the BET (bromodomains and extra-terminal) family provided compelling data supporting targeting of these BRDs in cancer. The BET (bromodomain and extraterminal domain) subfamily of bromodomain proteins, composed of BRD2, BRD3, BRD4, and BRDT, perform diverse roles in regulating transcription by RNA polymerase II (POLII) and are an exciting new class of epigenetic drug targets. These proteins facilitate the initiation and elongation phases of transcription by binding to activated chromatin at acetylated lysine residues. The recognition of activated chromatin by these so-called epigenetic "readers" promotes the recruitment of the RNA polymerase II complex to sites of active transcription. The BRD4/P-TEFb interaction is important for rapid transcriptional re-initiation after mitosis (Muller et al., 2011, Expert Rev. Mol. Medicine, 13, e19). P-TEFb was identified and purified as a factor needed for the generation of long run-off transcripts using an in vitro transcription system derived from Drosophila cells. It is a cyclin dependent kinase containing the catalytic subunit, Cdk9, and a regulatory subunit, cyclin T in Drosophila. In humans there are multiple forms of P-TEFb which contain Cdk9 and one of several cyclin subunits, cyclin T1, T2, and K. P-TEFb associates with other factors including the bromodomain protein BRD4, and is found associated with a large complex of proteins called the super elongation complex (Yang Z. et al., 2005. Mol Cell; 19:535-45; Fu et al., 1999, J Biol Chem., 274:34527-30).

JQ1 (thieno-triazolo-1-4-diazepine) is a potent inhibitor of the BET family of bromodomain proteins which include BRD2, BRD3, BRD4 (Filippakopoulos et al., 2010 Nature 468, 1067-1073). JQ1 prevent interaction between the bromodomain and the acetyl group, causing the downregulation of certain genes. Further BET bromodomain Inhibitors including OTOX15, BAY1238097, GSK2820151, I-BET762 and PLX51107 have been described (Perez-Salvia and Esteller 2017, EPIGENETICS, 12, 323-339; Brandt et al., 2015ACS Chem. Biol., 10, 22-39). JQ-1 is structurally related to benzodiazepines. The formula is C23H25ClN4O2S.

Recently, it has been shown that the BET inhibitor JQ1 facilitates adenovirus infection and adenoviral vector-mediated gene delivery. Treatment of cells with JQ1 induces an increase in BRD4 association with CDK9, a subunit of P-TEFb of transcription elongation. However, as stated in the paper, further studies are required to delicate the mechanism by which BED4 utilizes to regulate adenovirus infection and transgene expression (Baojie Lv et al. 2018, Scientific reports, 8, 11554). Importantly viral replication and virus transcription were not investigated. However, it is known, that CDK9 stimulates released of paused polymerase and activates transcription by increasing the number of transcribing polymerases and thus the amount of mRNA synthesis per time (Gressel et al. 2017, eLife, 6, e29736). In addition, it was shown, that BET inhibitor resistance can be overcome by CDK 4/6 inhibitors (Jin et al. 2018, Mol Cell;71(4):592-605). Recently it was demonstrated a dramatic increase in P-TEFb-Brd4 interaction from late mitosis to early G1 phases of cell cycle and active recruitment of P-TEFb to the chromosomes, followed by initiation of transcription of key genes for G1 progression. Importantly, depletion of Brd4 abrogated the whole process by reducing transcription of essential G1 genes, leading to G1 cell cycle arrest and apoptosis (Yang et al., 2008, Mol Cell Biol., 28:967-976, Kohoutek, 2009, Cell Division, 4. 19).

However, nothing is known about using YB-1 dependent oncolytic adenoviruses in conjunction with CDK 4/6 inhibitors and BET inhibitors.

It will be appreciated and is within the present invention that other bromodomain inhibitors will be equally suitable for use in triple therapy using a virus, preferably an adenovirus, more preferably an oncolytic adenovirus such as XVir-N-31, and a CDK4/6 inhibitor.

It will be appreciated by a person skilled in the art that any bromodomain (Bet) inhibitor is used in a therapeutically effective concentration.

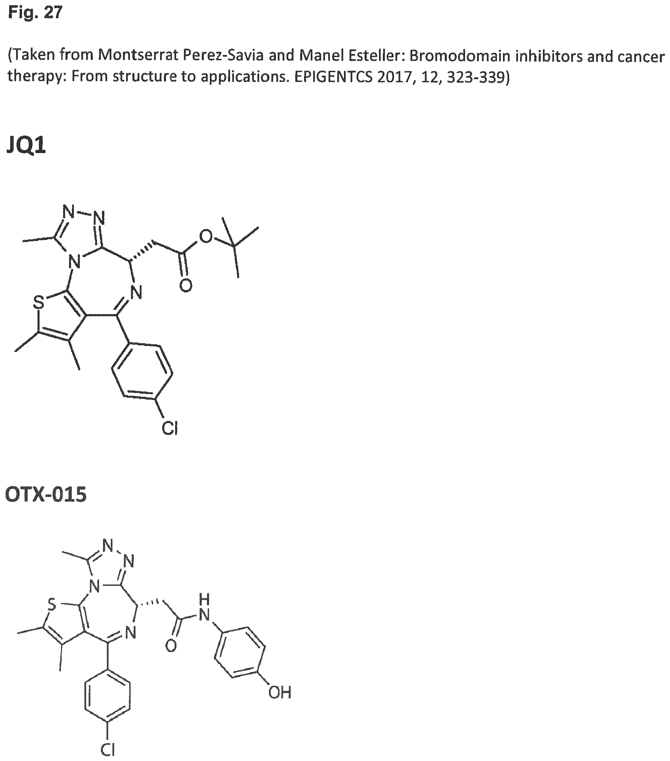

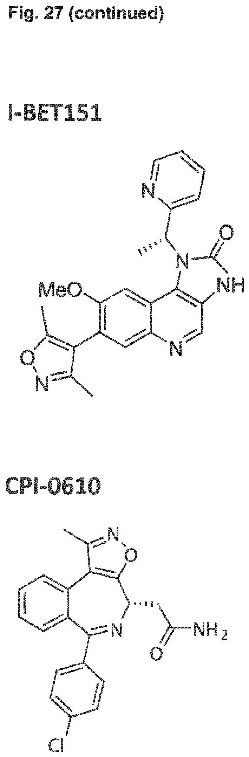

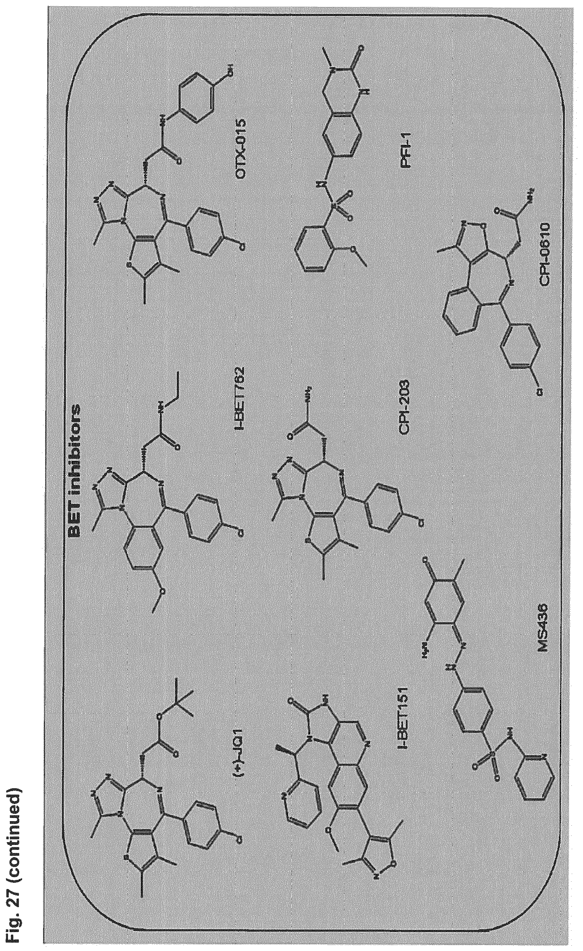

Bromodomain inhibitors suitable for use in the practicing of the present invention are disclosed in Fig. 27.

The tumours which can in particular be treated by the viruses and thus combinations of the present invention described herein are preferably those tumours which are selected from the group comprising tumours of the nervous system, ocular tumours, tumours of the skin, tumours of the soft tissue, gastrointestinal tumours, tumours of the respiratory system, tumour of the skeleton, tumours of the endocrine system, tumours of the female genital system, tumours of a mammary gland, tumours of the male genital system, tumours of the urinary outflow system, tumours of the haematopoietic system including mixed and embryonic tumours, and leukemia. It is within the present invention that these tumours are in particular resistant tumours as in particular defined herein.

The group of tumors of the nervous system preferably comprises:
1. Tumors of the skull as well as of the brain (intracranial), preferably astrocytoma, oligodendroglioma, meningioma, neuroblastoma, ganglioneuroma, ependymoma, schwannoglioma, neurofibroma, haemangioblastoma, lipoma, craniopharyngioma, teratoma and chordoma;
2. Tumors of the spinal cord and of the vertebral canal, preferably glioblastoma, meningioma, neuroblastoma, neurofibroma, osteosarcoma, chondrosarcoma, haemangiosarcoma, fibrosarcoma and multiple myeloma; and
3. Tumors of the peripheral nerves, preferably schwannoglioma, neurofibroma, neurofibrosarcoma and perineural fibroblastoma.

The group of the ocular tumors preferably comprises:
1. Tumors of the eyelids and of the lid glands, preferably adenoma, adenocarcinoma, papilloma, histiocytoma, mast cell tumor, basal-cell tumor, melanoma, squamous-cell carcinoma, fibroma and fibrosarcoma;
2. Tumors of the conjunctiva and of the nictitating membrane, preferably squamous-cell carcinoma, haemangioma, haemangiosarcoma, adenoma, adenocarcinoma, fibrosarcoma, melanoma and papilloma; and
3. Tumors of the orbita, the optic nerve and of the eyeball, preferably retinoblastoma, osteosarcoma, mast cell tumor, meningioma, reticular cell tumor, glioma, schwannoglioma, chondroma, adenocarcinoma, squamous-cell carcinoma, plasma cell tumor, lymphoma, rhabdomyosarcoma and melanoma.

The group of skin tumors preferably comprises:
Tumors of the histiocytoma, lipoma, fibrosarcoma, fibroma, mast cell tumor, malignant melanoma, papilloma, basal-cell tumor, keratoacanthoma, haemangiopericytoma, tumors of the hair follicles, tumors of the sweat glands, tumors of the sebaceous glands, haemangioma, haemangiosarcoma, lipoma, liposarcoma, malignant fibrous histiocytoma, plasmacytoma and lymphangioma.

The group of tumors of the soft-tissues preferably comprises:
Tumors of the alveolar soft-tissue sarcoma, epithelioid cell sarcoma, chondrosarcoma of the soft-tissue, osteosarcoma of the soft-tissues, Ewing's sarcoma of the soft-tissues, primitive neuroectodermal tumors (PNET), fibrosarcoma, fibroma, leiomyosarcoma, leiomyoma, liposarcoma, malignant fibrous histiocytoma, malignant haemangiopericytoma, haemangioma, haemangiosarcoma, malignant mesenchymoma, malignant peripheral nerve sheath tumor (MPNST, malignant schwannoglioma, malignant melanocytic schwannoglioma, rhabdomyosarcoma, synovial sarcoma, lymphangioma and lymphangiosarcoma.

The group of gastrointestinal tumors preferably comprises:
1. Tumors of the oral cavity and of the tongue, preferably squamous-cell carcinoma, fibrosarcoma, Merkel cell tumor, inductive fibroameloblastoma, fibroma, fibrosarcoma, viral papillomatosis, idiopathic papillomatosis, nasopharyngeal polyps, leiomyosarcoma, myoblastoma and mast cell tumor;
2. Tumors of the salivary glands, preferably adenocarcinoma;
3. Tumors of the oesophagus, preferably squamous-cell carcinoma, leiomyosarcoma, fibrosarcoma, osteosarcoma, Barrett carcinoma and paraoesophageal tumors;
4. Tumors of the exocrine pancreas, preferably adenocarcinoma; and
5. Tumors of the stomach, preferably adenocarcinoma, leiomyoma, leiomyosarcoma and fibrosarcoma.

The group of the tumors of the respiratory system preferably comprises:
1. Tumors of the nose and nasal cavity, of the larynx and of the trachea, preferably squamous-cell carcinoma, fibrosarcoma, fibroma, lymphosarcoma, lymphoma, haemangioma, haemangiosarcoma, melanoma, mast cell tumor, osteosarcoma, chondrosarcoma, oncocytoma (rhabdomyoma), adenocarcinoma and myoblastoma; and
2. Tumors of the lung, preferably squamous-cell carcinoma, fibrosarcoma, fibroma, lymphosarcoma, lymphoma, haemangioma, haemangiosarcoma, melanoma, mast cell tumor, osteosarcoma, chondrosarcoma, oncocytoma (rhabdomyoma), adenocarcinoma, myoblastoma, small-cell carcinoma, non-small cell carcinoma, bronchial adenocarcinoma, bronchoalveolar adenocarcinoma and alveolar adenocarcinoma.

The group of the skeleton tumors preferably comprises:
osteosarcoma, chondrosarcoma, parosteal osteosarcoma, haemangiosarcoma, synovial cell sarcoma, haemangiosarcoma, fibrosarcoma, malignant mesenchymoma, giant-cell tumor, osteoma and multilobular osteoma.

The group of the tumors of the endocrine system preferably comprises:
1. Tumors of the thyroid gland/parathyroid, preferably adenoma and adenocarcinoma;
2. Tumors of the suprarenal gland, preferably adenoma, adenocarcinoma and pheochromocytoma (medullosuprarenoma);
3. Tumors of the hypothalamus/hypophysis, preferably adenoma and adenocarcinoma;
4. Tumors of the endocrine pancreas, preferably insulinoma (beta cell tumor, APUDom) and Zollinger-Ellison syndrome (gastrin secernent tumor of the delta cells of the pancreas); and
5. as well as multiple endocrine neoplasias (MEN) and chemodectoma.

The group of the tumors of the female sexual system tumors preferably comprises:
1. Tumors of the ovaries, preferably adenoma, adenocarcinoma, cystadenoma, and undifferentiated carcinoma;
2. Tumors of the uterine, preferably leiomyoma, leiomyosarcoma, adenoma, adenocarcinoma, fibroma, fibrosarcoma and lipoma;
3. Tumors of the cervix, preferably adenocarcinoma, adenoma, leiomyosarcoma and leiomyoma;
4. Tumors of the vagina and vulva, preferably leiomyoma, leiomyosarcoma, fibroleiomyoma, fibroma, fibrosarcoma, polyps and squamous-cell carcinoma.

The group of tumors of the mammary glands preferably comprises:
fibroadenoma, adenoma, adenocarcinoma, mesenchymal tumora, carcinoma, carcinosarcoma.

The group of the tumors of the male sexual system preferably comprises:
1. Tumors of the testicles, preferably seminoma, interstitial-cell tumor and Sertoli cell tumor;
2. Tumors of the prostate, preferably adenocarcinoma, undifferentiated carcinoma, squamous-cell carcinoma, leiomyosarcoma and transitional cell carcinoma; and
3. Tumors of the penis and the external gentials, preferably mast cell tumor and squamous-cell carcinoma.

The group of tumors of the urinary outflow system preferably comprises:
1. Tumors of the kidney, preferably adenocarcinoma, transitional cell carcinoma (epithelial tumors), fibrosarcoma, chondrosarcoma (mesenchymal tumors), Wilm's tumor, nephroblastoma and embryonal nephroma (embryonal pluripotent blastoma);
2. Tumors of the ureter, preferably leiomyoma, leiomyosarcoma, fibropapilloma, transitional cell carcinoma;
3. Tumors of the urinary bladder, preferably transitional cell carcinoma, squamous-cell carcinoma, adenocarcinoma, botryoid (embryonal rhabdomyosarcoma), fibroma, fibrosarcoma, leiomyoma, leiomyosarcoma, papilloma and haemangiosarcoma; and
4. Tumors of the urethra, preferably transitional cell carcinoma, squamous-cell carcinoma and leiomyosarcoma.

The group of tumors of the haematopoietic system preferably comprises:
1. Lymphoma, lymphatic leukemia, non-lymphactic leukemia, myeloproliferative leukemia, Hodgkin's lymphoma, Non-Hodgkin's lymphoma.

The group of the mixed and embryonal tumors preferably comprises:
Haemangiosarcoma, thymoma and mesothelioma.

Preferably, these tumors are selected from the group comprising breast cancer, ovary carcinoma, prostate carcinoma, osteosarcoma, glioblastoma, melanoma, small-cell lung carcinoma and colorectal carcinoma. Further tumors are those which are resistant as described herein, preferably those which are multiple resistant, particularly also those tumors of the group described above.

Subjects to which the combination of the invention as defined in the claims is to be administered are identified and screened, respectively. Such identification of patients who may benefit from the present invention in its diverse aspects, is based on the detection of YB-1 in the nucleus of a sample of a subject.

The examination of the tumor tissue may be done by using an agent which is selected from the group comprising antibodies against YB-1, aptamers against YB-1 and spiegelmers against YB-1 as well as anticalines against YB-1. Basically, the same means can be produced for the corresponding markers and used accordingly. The manufacture of antibodies, in particular monoclonal antibodies, is known to the ones skilled in the art. A further means for specific detection of YB-1 or the markers, are peptides which bind with a high affinity to the target structures, in the present case YB-1 or said markers. In the prior art methods are known such as phage-display in order to generate such peptides. Typically, a peptide library is taken as a starting point, whereby individual peptides have a length of from 8 to 20 amino acids and the size of the library is about 102 to 1018, preferably 108 to 1015 different peptides. A special form of target molecule binding polypeptides are the so-called anticalines which are, for example, described in German patent application DE 197 42 706.

A further means for specific binding of YB-1 or the corresponding markers disclosed herein and thus for the detection of cell cyclus independent localisation of YB-1 in the cellular nucleus, are the so-called aptamers, i.e. D-nucleic acids which are present either as RNA or DNA either as a single strand or a double strand and specifically bind to the target molecule. The generation of aptamers is, for example, described in European patent EP 0 533 838. A special form of aptamers are the so-called aptazymes, which, for example, are described by Piganeau, N. et al. (2000), Angew. Chem. Int. Ed., 39, no. 29, pages 4369 - 4373. These are special forms of aptamers insofar as they comprise apart from the aptamer part a ribozyme part and get catalytically active upon binding or release of the target molecule binding to the aptamer part and cleave a nucleic acid substrate which goes along with the generation of a signal.

A further form of aptamers are the so-called spiegelmers, i. e. target molecule binding nucleic acids which are made of L-nucleic acids. The method for the manufacture of such spiegelmers is, for example, described in WO 98/08856.

The sample of the tumor tissue can be obtained by puncture or through surgery. The assessment whether YB-1 is localised in the nucleus independent from the cell cycle, is frequently done by using microscopic techniques and/or immuno histoanalysis, preferably using the antibody or any of the other aforementioned means. Further means for detecting YB-1 in the nucleus and in particular for detecting that YB-1 is located there independent from the cell cycle, are known to the one skilled in the art. For example, the localisation of YB-1 can be easily detected in stained tissue sections when screening them. The frequency of the presence of YB-1 in the nucleus already indicates that the localisation is independent from the cell cycle. A further option for cell cycle independent detection of YB-1 in the nucleus resides in the staining against YB-1 and detection whether YB-1 is localised in the nucleus and determination of the phase of the cells. This as well as the detection of YB-1 may also be performed by using the afore-mentioned means directed against YB-1. The detection of the means is done by methods known to the one skilled in the art. By said agents specifically binding to YB-1 and not to any other structures within the sample to be analysed, particularly the cells, their localisation and because of their specific binding to YB-1 also the localisation of YB-1 can be detected and established by a suitable labelling of the means. Methods for the labelling of said means are known to the ones skilled in the art.

In the following, the present invention shall be further illustrated by reference to the figures and samples from which new features, embodiments and advantages may be taken.
Fig. 1 a is a bar diagram showing relative absorbance as an indicator of cell viability for XVir-N-31 (XVir), wild type adenovirus (WT) and control (Ctrl) when used in combination with CDK4/6 inhibitors LY (LY-2835219), PD (PD-032991) or LEE (LEE011).
Fig. 1b is a bar diagram showing viral titre for XVir-N-31 (XVir) and wild type adenovirus (WT) when combined with CDK4/6 inhibitors LY (LY-2835219), PD (PD-032991) or LEE (LEE011).
Fig. 1c is a bar diagram showing relative fiber DNA for XVir-N-31 (XVir) and wild type adenovirus (WT) when combined with CDK4/6 inhibitors LY (LY-2835219), PD (PD-032991) or LEE (LEE011).
Fig. 2 depicts the result of a Western blot analysis.
Figs 3a-d are bar diagrams.
Figs 4a-d are bar diagrams.
Fig. 5 are bar diagrams.
Fig. 6 is a series of microphotographs.
Fig. 7 is a fluorescence microscopic image of T24 cells infected with an E1-deleted Adenovirus expressing GFP with and without Palbociclib treatment.
Fig. 8 is a bar diagram showing viral DNA replication of Adenovirus dl703 after 48 h using compounds Nutlin 3a, Lee, Cl1040 and Roscovertine.
Figs 9A - C show the result of a Western blot analysis of UMUC cells treated with indicated concentrations of Nutlin-3a and LEE011 (Ribociclib) (Fig. 9A), Roscovitine (Fig.
9B) and CI-1040 (Fig. 9C); Rb means retinoblastoma protein; phRB means phosphorylated retinoblastoma protein; E2F-1 means transcription factor E2F-1; and GAPDH served as loading control.
Fig. 10 is a bar diagram showing cell cycle distribution in UMUC3 cells, measured 48 hours post treatment, whereby the concentrations of the CDK4/6 inhibitors was as follows: Roscovetine: 10 µM, CI-1040: 1 µM, Nutlin-3a: 10 µM, and LEE011: 10 µM.
Fig. 11 is a panel of microscopic images showing adenovirus hexon gene expression with and without Palbociclib treatment.
Fig. 12 is a bar diagram showing the result of a potency assay of T24 cells exposed to XVir-N-31 alone, with 15 nM PARP inhibitor PARPi, 500 nM PD (Palbociclib) or a combination of 15 nM PARPi and 500 nM PD, as percentage cell survival, whereby the cells were either not infected (left column), infected with an MOI of 10 (middle column) or an MOI of 50 (right column).
Fig. 13 is a panel of pictures showing cultures of SRB-stained T24 cells after treatment with XVir-N-31 (20 MOI), XVir-N-31 and 15 nM PARPi, XVir-N-31 and 500 nM PD, and XVir-N-31, 15 nM PARPi and 500 nM PD, 1 dpi, 2 dpi, 3 dpi, 4 dpi, 5 dpi and 6 dpi.
Fig. 14 is a panel of pictures showing cultures of SRB-stained UMUC cells after treatment with XVir-N-31 (10 MOI), XVir-N-31 and 160 nM PARPi, XVir-N-31 and 400 nM PD, and XVir-N-31, 160 nM PARPi and 400 nM PD, 1 dpi, 2 dpi, 3 dpi, 4 dpi, 5 dpi and 6 dpi.
Fig. 15 is a bar diagram showing the result of a potency assay on T24 cells 5 days post infection with XVir-N-31, the CDK 4/6 inhibitor Palbociclib and the bromodomain inhibitor JQ-1; Y-axis: survival of cells in %.
Fig. 16 is a bar diagram showing the result of a potency assay of SK-N-MC cells 5 days after exposure to XVir-N-31 alone, with 200 nM abemaciclib, 500 nM JQ1 or a combination of 200 nM abemaciclib and 500 nM JQ1, as percentage cell survival, whereby the cells were either not infected or infected with an MOI of 5, 10 or 20.
Fig. 17 shows the result of a Western blot analysis of SK-N-MC cells treated with indicated concentrations of CDK 4/6 Inhibitor LY-2835219 (Abemaciclib) and the Wee-Inhibitor MK-1775 (Adavosertib) 24 and 48 hours post treatment; Rb means retinoblastoma protein; phRB means phosphorylated retinoblastoma protein; E2F-1 means transcription factor E2F-1; and GAPDH served as loading control.
Fig. 18 shows the result of a potency assay on SK-N-MC cells 5 days post infection with XVir-N-31, the CDK 4/6 inhibitor Abemaciclib and Adavosertib (Wee-inhibitor MK-1775) expressed as percentage of living cells.
Fig. 19 shows cell cycle distribution after treatment of SK-N-MC cells with the indicated inhibitors.
Fig. 20 is a bar diagram showing the effect of E2F1 directed siRNA on E2F1 expression in various cell lines; Y axis: E2F1 expression normalized to actin as % of siCTRL transfected cells.
Fig. 21 is a bar diagram showing that E2F1 inhibition causes increased E2-early expression in T24 cells treated with siRNA-E2F-1; Y axis: adenoviral gene expression normalized to actin (in % of siCTRL).
Fig. 22 is a scheme showing location of the primers for determining adenovirus E2-early expression.
Fig. 23 is a representation of the nucleotide sequence of the wild type E2 early promoter adenovirus (above) and a mutant E2 early promoter having mutations at the E2F-binding sites (below).
Fig. 24 is a bar diagram showing RNA expression in AdWT-RGD and AdE2Fm (also containing the RGD motive in the fibre) infected T24 cells obtained by RT-qPCR at 24 hours post infection; AD-WT gene expression was set to 100%.
Fig. 25 shows various CDK4/6 inhibitors suitable for use in the instant invention.
Fig. 26 shows various PARP inhibitors suitable for use in the instant invention.
Fig. 27 shows various Bet inhibitors suitable for use in the instant invention.

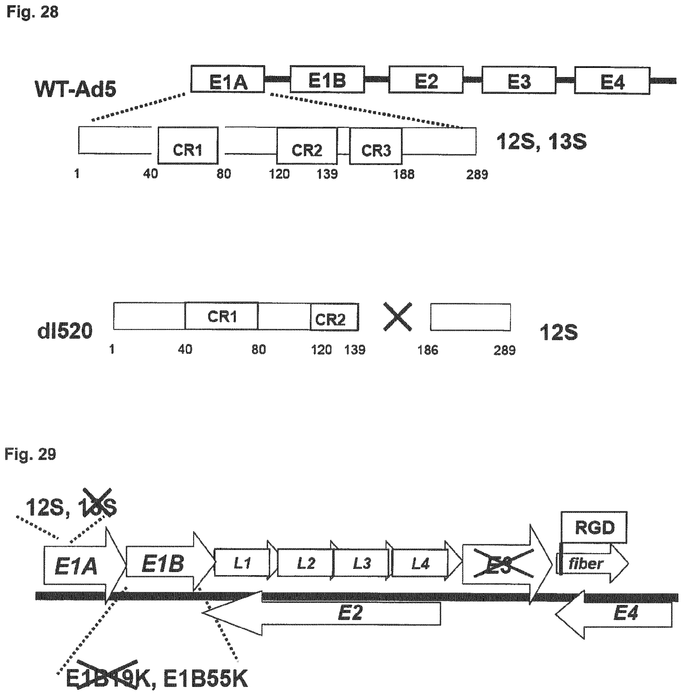

Fig. 28 shows the structure of WT-Ad5 and adenovirus dl520 which is an oncolytic adenovirus expressing only the E1A12 protein, through deletion of the CR3-domain of the E1A gene.
Fig. 29 shows the structure of XVir-N-31 which is characterized by deletion of the E1B19K protein, deletion of 2 kb in E3-region, deletion des E1A13S Protein, and introducing a RGD motif the fiber protein.
Fig. 30 shows the structure of Ad-Delta 24 and Ad-Delta 24-RGD which are also described by Kleijn et al. (Kleijn et al., PLoS One. 2014; 9(5): e97495), and characterized by deletion of the CR2-domain of the E1A gene; it replicate only in tumor cells with deregulated retinoblastoma-pathway (Rb). Ad-Delta 24-RGD contains in addition a RGD motive in the fiber knob, as shown in XVir-N-31. Please note the oncolytic adenovirus dl922-947 is similar to delta24, since the deletion in this virus also is located in the E1A-CR2 domain and is affecting RB-binding (retinoblastoma protein).

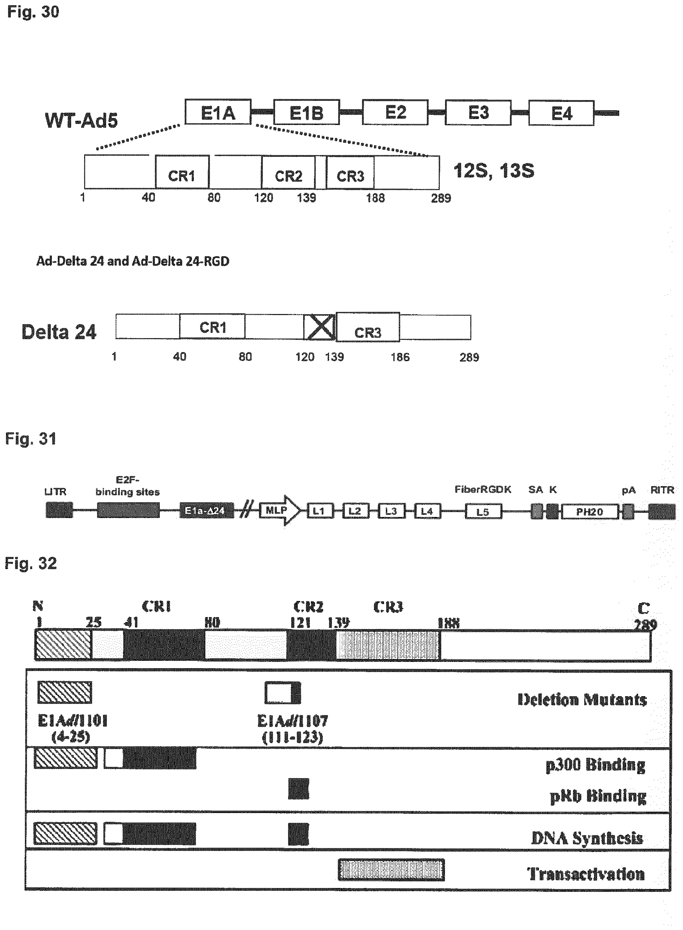

Fig. 31 shows the structure of VCN-01 which is a replication-competent adenovirus specifically engineered to replicate in tumors with a defective RB pathway, presents an enhanced infectivity through a modified fiber and an improved distribution through the expression of a soluble hyaluronidase (Pascual-Pasto et al. Sci Transl Med. 2019,11 476). The deletion in E1A in VCN-01 is similar to the deletion in delta24 (deletion of the CR2-domain in E1A). Further, the expression of this E1A protein is regulated by introducing E2F binding sites in the E1A promoter. In addition, it contains an RGD motif in the fiber knob and expresses a soluble hyaluronidase (Martínez-Vélez et al. 2016, Clin Cancer Res. 1;22(9):2217-25. The Oncolytic Adenovirus VCN-01 as Therapeutic Approach Against Pediatric Osteosarcoma).
Fig. 32 shows the structure of E1Adl1107 and E1Adl1101, whereby the deletion of these two oncolytic adenoviruses affects binding to p300 (Histone acetyltransferase p300 also known as p300 HAT or E1A-associated protein p300) or pRb (retinoblastoma protein. (Howe et al., MOLECULAR THERAPY 2000, 2, 485-495)

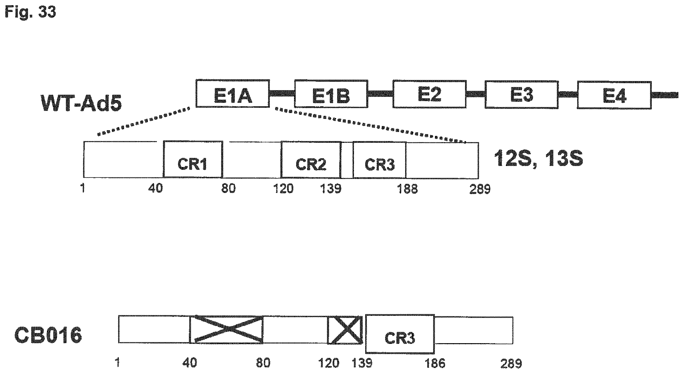

Fig. 33 shows the structure of oncolytic adenovirus CB016 (and the one of wild type adenovirus 5 (WT-Ad5), where deletion in the E1A-CR2 domain is similar as in Ad-Delta 24. In addition, CB016 contains a deletion in the CR1 domain. In addition, it contains either an RGD motif in the fiber or a fiber from serotype 3 (LaRocca et al., Oral Oncol. 2016, 56, 25-31).

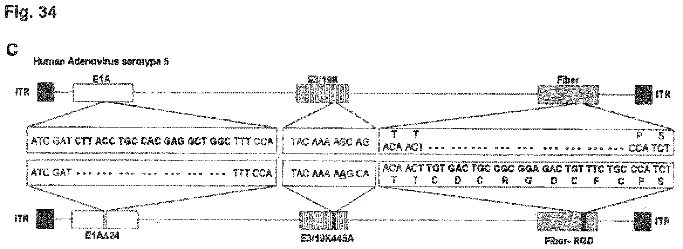

Fig. 34 shows the structure of adenovirus ORCA-010 which contains an E1AΔ24 deletion in the E1A CR2-domain, the potency-enhancing T1 mutation in the E3/19K protein, and the infectivity-enhancing fiber RGD modification (Dong et al., Hum Gene Ther. 2014 Oct 1; 25(10): 897-904).

### Example 1: Materials and Methods

### Cell Culture

Human bladder cancer cell lines were cultured under subconfluent conditions in RPMI or DMEM medium (Biochrom AG) at 5% or 10% CO2, respectively, supplemented with 10% FBS (Biochrom AG) and 1% NEA (Biochrom AG). Depending on the cell line and experimental conditions, 0.2-1x106, 0.5-1x105, 0.25-0.5x105, and 500-700 cells were seeded in 10 cm, 6-well, 12-well and 96-well formats, respectively.

### Cell lines

### HeLaP

HeLa P cells (ATCC CCL-2) are epithelial cells from cervical adenocarcinoma named after the patient Henrietta Lacks. This cell line is the most widely distributed and oldest cell line (Rahbari et al., 2009), since it was the first permanent cell line, established in 1951 (Gey et al., 1952). Cultivation occurred in DMEM (10% FBS, 1% PS) under 10% CO2 conditions at 37°C.

### HeLaRDB

HeLaRDB is a sub-cell line of the HeLaP-cell line, with resistance to daunoblastin based on overexpression of the glycoprotein P. The resistance was achieved through cultivation with medium containing this anthracycline. This cytostatic agent intercalates in double-stranded DNA sequences and inhibits cellular transcription and replication (Mizuno et al., 1975). As a result of the stress reactions, caused by daunoblastin treatment, the cellular factor YB-1 shows higher nuclear localization in comparison to the parental cell line (Holm et al., 2004). To maintain the resistance against daunoblastin, the cells were cultured in DMEM (10% FBS, 1% PS) containing 0.25 µg/ml daunoblastin under 10% CO2 conditions at 37°C every 14 days.

### A549

A549 cells (ATCC CCL-185) were isolated in 1972 from an adenocarcinoma in the human alveolar basal (Giard et al., 1973). Cultivation occured in Dulbecco's MEM (10% FBS and 1% PS) at 37 °C and 10% CO2.

### T24

T24 cells (ATCC HTB-4) derived 1970 of a primary human urinary bladder carcinoma (Bubenik, Baresovà et al., 1973). Due to a point mutation in the HRAS gene (Reddy et al., 1982), the MAPK and PI3K pathway is activated. Moreover, an additional mutation in the gene locus of the tumor suppressor gene p53 is present in this cell line (Pinto-Leite et al., 2014). The cells were cultivated with RPMI containing 10% FCS, 1% PS and 1% non-essential amino acids at 37°C under 5% CO2 conditions.

### HEK293

HEK293 cells (ATCC CRL-1573) are human embryonic kidney cells isolated in 1973. Due to a stabile transfection of a 4.5 kb-sized part of the genome of adenoviral serotype 5, which includes the whole E1 region (Graham and Smiley, 1977), this cell line is used for production of E1-deficient adenoviruses and for measurement of virus titer.

**Table 1: Primer**

| Name Company | Forward primer | Reverse primer |
|---|---|---|
| Fiber Eurofins | AAGCTAGCCCTGCAAACATCA CCCAAGCTACCAGTGGCAGTA (SEQ ID NO: 1) | |
| E2 early Invitrogen | CCGTCATCTCTACAGCCCAT GGGCTTTGTCAGAGTCTTGC (SEQ ID NO: 2) | |
| E2 late Invitrogen | CTTCCTAGCGACTTTGTGCC GTCAGAGTGGTAGGCAAGGT (SEQ ID NO: 3) | |
| E1A 12S Metabion | CGACGAGGATGAAGTCCTGTGTCTG CTCAGGATAGCAGGCGCCAT (SEQ ID NO: 4) | |
| E1A 12S short Metabion | GAGGATGAAGTCCTGTGT CTCAGGATAGCAGGCGCCAT (SEQ ID NO: 5) | |
| E1A 13S Metabion | TGTTTGTCTACAGTCCTGTGTCTG CTCAGGATAGCAGGCGCCAT (SEQ ID NO: 6) | |
| E1A 13S short | | |
| Metabion | TTGTCTACAGTCCTGTGT CTCAGGATAGCAGGCGCCAT (SEQ ID NO: 7) | |
| E4orf6 Metabion | TCCCTCCCAACA CACAGAGT GACAGGAAACCG TGTGGAAT SEQ ID NO: 8) | |
| Rb Life Techno. | AGCAACCCTCCTAAACCACT TGTTTGAGGTATCCATGCTATCA (SEQ ID NO: 9) | |
| E2F1 Life Technology | ACGCTATGAGACCTCACTGAA TCCTGGGTCAACCCCTCAAG (SEQ ID NO: 10) | |
| E2F2 Life Technology | CGTCCCTGAGTTCCCAACC GCGAAGTGTCATACCGAGTCTT (SEQ ID NO: 11) | |
| GAPDH MWG | TGGCATGGACTGTGGTCATGAG ACTGGCGTCTTCACCACCATGG (SEQ ID NO: 12) | |
| Actin Eurofins | TAAGTAGGTGCACAGTAGGTCTGA AAAGTGCAAAGAACACGGCTAAG (SEQ ID NO: 13) | |
| L4 33K Eurofins | GAACCAGGGCCGCCCATACTG GGGCTTTGTCAGAGTCTTGC (SEQ ID NO: 14) | |
| L4 22 K Eurofins | CCGTTAGCCCAAGAGCAAC CGGCCGTGATGGTAGAGAAG (SEQ ID NO: 15) | |
| L4HexAss Eurofins | CTGTGGTACTTCCCAGAGAC CAGGTGAGTTATACCCTGCC (SEQ ID NO: 16) | |

### Virus Characteristics

| | | |
|---|---|---|
| Ad-WT +AdWT-RGD | Wildtype Mastadenovirus, Type C, Serotype 5 and ADWT with additional RGD-fiber motif | |
| AdWT-E2Fmut. | Mastadenovirus, Type C, Serotype 5, mutations in both E2F binding sites of the E2-early promoter with additional RGD-fiber motif and a 2,7 kb-sized deletion in the E3 region (ΔE3) | |
| XVir-N-31 | Mastadenovirus, Type C, Serotype 5 with deletions in the E1B-region (1.716-1915, 200 bp), E3-region (28.132-30.813) and 12 base deletion in the E1A-region. Replicates in cancer cells only displaying nuclear YB-1 expression. | |
| XVir-N-31/E2FM | Mastadenovirus, Type C, Serotype 5 with deletions in the E1B-region (1.716-1915, 200 bp), E3-region (28.132-30.813) and 12 base deletion in the E1A-region. Replicates in cancer cells only displaying nuclear YB-1 expression. mutations in both E2F binding sites of the E2-early promoter with additional RGD-fiber motif and a 2,7 kb-sized deletion in the E3 region (ΔE3) | |
| Target gene | siRNA construct | Manufacturer |
| Control | Control (non-sil.) siRNA, 20 µM | Qiagen, the Netherlands |
| E2F-1 | E2F-1 (SASI_Hs01_00162220), 10 µM | Sigma, Merck, Germany |
| YB-1 | YBX1 siRNA FlexiTube, 10µM | Qiagen, the Netherlands |

### Methods

### siRNA Transfection

Downregulation of certain genes was performed using siRNA transfection. Thereby, 5 µl Lipofectamin RNAiMAX (Thermo Fischer) reagent was added to 150 µl of Opti-MEM in one tube and 36 pmol of siRNA was combined with 150 µl of Opti-MEM in another tube. After combining the contents of both tubes and brief vortexing, the solution was incubated for 5 minutes at room temperature. 250 µl of the siRNA-lipid complex was then added to the 250.000 - 1.000.000 cells, seeded in 6-well plates on the previous day without changing the medium, reaching a final concentration of siRNA of 30 pmol per well. After 48 hours of incubation at 37°C at 10% CO2 conditions, infection or lysation took place.

### RNA-Quantification in combination with siRNA

RNA was also quantified in cells where virus was combined with siRNA transfection. Thereby, 125.000 cells were seeded and transfected on the following day with 30 pmol of siRNA-construct of Ctrl-, YB-1-, and E2F1-siRNA. After 48 hours of incubation, infection took place and lysation occurred 24 hours post infection. The lysates were stored at -20°C.

### RNA Isolation

Cells were rinsed with PBS and lysed with lysis buffer (mirVana miRNA isolation kit, Life Technologies) and transferred into 1.5 ml reaction tube. 50 µl of homogenate additive (mirVana miRNA isolation kit, Life Technologies) was added to the lysates, resuspended and incubated for 10 minutes on ice. 500 µl of Acid-Phenol-Chloroform was added, vortexed for approximately 30 seconds and incubated for 2 minutes on ice. After centrifugation for 5 minutes at room temperature at 14.000 g, the aqueous and organic phases are separated. The upper aqueous phase was transferred to a new snap cap and combined and inverted with the equal amount of Isopropanol. After incubation for 10 minutes at room temperature the samples were centrifuged at 4°C and 14.000 g for 30 minutes. Subsequently, the supernatant was removed and the RNA pellet was washed with 1 ml 75% ethanol. The samples were briefly centrifuged at 7500 g for 5 minutes at 4°C. After removing the supernatant, the air-dried pellet was solved in 20 µl nuclease-free water and incubated for 10 minutes at 55°C and 500 rpm in a thermomixer. Subsequently the RNA concentrations were measured via spectrophotometral measurement. To avoid amplification of ruts of DNA, a DNAse digestion was performed. Thereby the Deoxyribonuclease I, Amplification Grade-Kit by Invitrogen by life technologies was used. To 1 µg RNA, 1 µl 10x DNAse I Reaction buffer and 1 µl DNAse I are added and filled with DEPC-treated water to an end volume of 10 µl and incubated for exactly 15 minutes at room temperature. By adding 1 µl of 25 mM EDTA solution, the DNase I is inactivated and thereby the process of DNAse digestion is stopped. The samples were incubated for 10 minutes at 65°C and were then used for reverse transcription.

### Reverse Transcription

To rewrite RNA to cDNA the High capacity cDNA Reverse Transcription Kit (Thermo Scientific) was used. 2 µg RNA of the DNA digested samples were added to Mastermix containing transcription buffer, 100 mM dNTPs and RNAse inhibitor in PCR soft tubes. Thereby it had to be considered, that the RNA transcribed via the E2-early and E2-late promoter could not be rewritten by random primers, usually used for reverse transcription, because these random primers would bind to both strands of the double-stranded adenoviral genome. Therefore, the rewriting from RNA to cDNA for the samples used for the E2-early and E2-late quantification was performed by using the specific E2-early reverse primer (Table 1). For the housekeeping gene actin, that was used to normalize the results, the random primer was used.

### DNA-Replication Analysis

To investigate viral replication within infected cells DNA-replication analysis was performed. 125.000 cells were seeded in 6-well plates and infected with 10-20 MOI. After 2 respectively 8, 12, 24, 36 and 48 hours post infection, lyzation took place. Thereby, the medium was removed, and the adherent cells were washed with 1 ml PBS. After adding 200 µl DNA-lysis buffer, the adherent cells were detached from the plate using a cell scraper. The lysate was then transferred into a snap cap. 3 µl of the enzyme proteinase K was added and incubated at the 56°C and 550 rpm at a thermomixer overnight. On the following day, DNA isolation was performed.

### DNA Isolation

For purification of DNA, 200 µl Phenol-Chloroform-Isoamylalcohol was added to the lysate. After vortexing and subsequent incubation for 5 minutes on ice, a phase separation was achieved by centrifugation for 3 minutes at 16430 g at 4°C. The upper aqueous phase was transferred to a new snap cap, containing 200 µl Chloroform and 20 µl cresol red in 10 mM TrisCl for a better visualization of the phases. After vortexing and incubation for 5 minutes for 5 minutes on ice, centrifugation for 3 minutes at 16430 g at 4°C took place. Again, the upper aqueous phase was combined with 800 µl ethanol and 50 µl 3M sodium acetate solution. 2 µl Glycogen was added, to achieve a better precipitation. After short inversion of the tube, the solution was centrifuged for 30 minutes at 16430 g at 4°C. Subsequently, the DNA pellet was covered by 400 µl 70% ethanol and incubated for 10 minutes at room temperature. After centrifugation for 7 minutes at 4760 g at room temperature, the DNA pellets were dried for about 5-10 minutes at 37°C. Subsequently, the pellets were dissolved in 100 µl 0,1×TE-buffer and shaken at 40°C at 400 rpm for approximately 3 hours. When the DNA was completely dissolved, the DNA concentration was measured by means of a spectrophotometer using 2 µl of DNA solution for the measurement and 0,1×TE-buffer as a blank solution. The DNA was then stored at 4°C.

### qPCR

For further quantification real time quantitative PCR were used. 5 µl of Template DNA respectively cDNA was used in a final concentration of 10 ng/µl. qPCR was performed using 10 µl Mastermix GoTaq qPCR (Promega Corporation) (7,5 µl Mastermix, 1,5 µl primer, 1 µl H2O) and 5 µl DNA template in a 96-well plate pipetted as duplicates. Relative quantification was performed using the comparative CT method with two normalizer genes. The plate was closed via a foil and centrifuged at room temperature for 2 minutes at 220 g. Then the plate was incubated following a certain temperature-time-program in the thermal cycler. Primer used are listed in table 1. Reactions were carried out on a CFX96 Real-Time PCR detection system (Bio-Rad Laboratories).

### qPCR cycling conditions

Fiber: 94°C for 2 minutes, 94 °C for 15 seconds, 60 °C for 15 seconds and 72 °C for 15 seconds, for 45 cycles
Other viral genes: 94°C for 1,5 minutes, 94 °C for 15 seconds, 58 °C for 15 seconds and 72 °C for 15 seconds, for 45 cycles
Rb: 94°C for 2 minutes, 94 °C for 15 seconds, 60 °C for 30 seconds and 72 °C for 1 minute, for 44 cycles
E2Fs: 95°C for 2 minutes, 95 °C for 15 seconds, 60 °C for 30 seconds and 72 °C for 30 seconds, for 40 cycles

### Protein isolation

Cells were lysed using an 1% SDS buffer, to achieve the disruption of the nuclear membrane. To avoid denaturation of the proteins, the whole process was performed on ice. After suctioning the medium, the cells were washed twice with cold PBS. The adherent cells of one well of a duplicate approach was lysed with 200 µl of 1% SDS buffer and scraped by means of a cell scraper. The lysate was then transferred to the other well of the duplicate approach and again scraped. The lysate of both wells combined, was then transferred in a snap cap tube. Subsequently the lysates were treated with a syringe, to destroy the viscous DNA and centrifuged for 30 minutes at 4°C with 31000 rpm. Because the proteins are present in the supernatant, the supernatant was transferred into a new snap cap tube and used for further steps.

### Protein quantification

To quantify the amount of protein, the bicinchoninic acid (BCA) assay by means of the Pierce TM BCA Protein Kit was performed. Thereby 112,5 µl of the BCA solution A+B (50:1) and 12,5 µl of the sample were added into one well of a 96-well plate and incubated for 30 minutes at 37°C. Dependent on the protein concentration, a staining of the solution resulted. By means of a standard series with known protein concentrations, the protein concentrations of the samples were determined by photometric measurement at 562 nm in the microplate reader.

### SDS gel electrophoresis

To separate the proteins in subsequent sodium dodecyl sulfate polyacrylamide gel electrophoresis, the calculated amounts of lysate and lysis buffer were mixed with 15 µl loading buffer-DDT-Mixture (6:1). The protein loading substances were then cooked for five minutes at 100°C. 5 µl of the color protein standard and 40 µl of the samples were then loaded onto the gel. For protein separation with detection of viral proteins a 10% gel was used. To study the downregulated genes via siRNA, 12% gels were used. The composition of the resolving and stacking gels are listed in section Buffers and solutions. For approximately 20 minutes the gel was running in TGS-Buffer at 90 V to concentrate all proteins in one band. Subsequently the gels run for approximately 60 minutes at 150 V in TGS-Buffer, to separate the proteins by size.

### Western Blot

To transfer the proteins from the gel onto a membrane it was blotted using the western blot technique. To activate the hydrophobic PVDF-membrane, it was incubated for about 2 minutes in methanol. Subsequently, the membrane together with the sponges, filter papers and the gel were deposit in blotting buffer. By means of electrophoresis for approximately two hours at 100V at 4°C, the proteins were transferred on the membrane in blotting buffer. To avoid unspecific antibody binding, the membrane was blocked rotating for one hour at room temperature in 10 ml 5% milk powder in TBST for analyzing cellular proteins respectively in 5 ml 5% BSA-TBST for the subsequent use of antibodies detecting viral proteins. After washing the membrane five times in TBST for five minutes each, the membrane was incubated with the primary antibody solution at 4°C rotating overnight. For the antibodies GAPDH, E1A, E1B55K, E2A and E4orf6 this step was performed for one hour at room temperature. The antibodies were thereby diluted with different factors in 5% BSA in TBST with 0,02% sodium azide. After additional five washing steps, the membrane was incubated rotating for 30 Minutes at room temperature in a 1:10.000 dilution of the secondary antibody. The secondary antibody (anti-mouse) for the viral antibodies were diluted in 5% BSA-TBST, all others in 5% milk powder in TBST. Those secondary antibodies are conjugated with a horse-radish peroxidase. After five final washing steps, the membrane was incubated five minutes in Enhanced-Chemi-Luminescence (ECL) solution to visualizing the signal of the peroxidase. For the membranes, incubated with the primary antibodies DP-1 and E2F-1 the Amersham ECL Prime Western Blotting Detection Reagent by GE-Healthcare was used to achieve brighter signals, for all others, ECL solutions produced in the lab were used. The composition of ECL A and ECL B, that are mixed shortly before usage 1: 1 are listed in section Buffers and solutions. Finally, the proteins could be detected by means of developing the signal on a film.

### Antibodies:

Checkpoint kinase 1 (sc-377231, Santa Cruz Biotechnology)
total RB (554136, BD Biosciences)
phospho RB Ser 780 (8180, Cell Signaling Technology)
E2F1 (sc-251, Santa Cruz Biotechnology)
E2F2 (ab138515, abcam)
E2F3 (PG37, Thermo FisherScientific)
E2F4 (WUF10, Thermo Fisher Scientific)
E2F5 (sc-999, Santa Cruz Biotechnology)
cyclin D1 (92G2, Cell Signaling Technology)
cyclin E2 (4132, Cell Signaling Technology)
CDK2 (78B2, Cell Signaling Technology)
GAPDH (14C10, Cell Signaling Technology)
actin (A2066, Sigma-Aldrich Chemie GmbH)
E1A (sc-25, Santa Cruz Biotechnology)
E1B55k (kindly provided by M. Dobbelstein)
E4orf6 (kindly provided by M. Dobbelstein
E2A (DBP, kindly provided by M. Dobbelstein)
Hexon (ABIN2686029, Antibodies online)

### Small molecule inhibitor treatment

PD-0332991 isethionate (Palbociclib, Sigma-Aldrich Chemie GmbH) and LY-2835219 (Abemaciclib, Selleck Chemicals) were dissolved in sterile water as 10mM stock solution. LEE011 (Ribociclib, MedChem Express) and Nutlin-3a (Sigma) was dissolved in DMSO as 10mM and 5µM stock solution, respectively. Working concentrations were prepared freshly for immediate use.

### Virus infection and combination treatment

For determination of virus induced cell killing, cells were seeded in 12-well plates. For combination treatment with PD-033299, LY-2835219, and LEE011, cells were pretreated with the inhibitors for 24 h. Cells were infected with the indicated viruses at indicated MOI in 200-400µl medium without FBS. At 1 hpi, complete medium with or without small molecule inhibitors was added to the cells.

### Cell viability (SRB Assay)

Cells were fixed with 10% TCA for 1 h at 4°C and stained with 0.5% sulforhodamine B (SRB, Sigma-Aldrich Chemie GmbH) in 1% acetic acid for 30 min at RT, followed by washing with 1% acetic acid to remove excess of SRB. Dried SRB was dissolved in 10mM Tris buffer and quantification was performed by photometric measurement at 590nm.

### Titer test

For determination of infectious viral particle production, infected cells and supernatant were harvested three dpi using cell scrapers. Virus was released from intact cells by multiple cycles of freeze-thaw followed by centrifugation at 1600 rcf. Supernatants of the cell lysates were tested for viral particle production using Hek293 cells as described in AdEasy Viral Titer Kit instruction manual (972500). The following reagents were used: goat-anti-hexon antibody (1056, Chemicon), rabbit-anti-goat antibody (P0449, Dako), DAB solution (Dako).

### Example 2: Effect of CDK4/6 inhibitor PD0332991 on replication of an E1-minus adenovirus

It was shown that E1-deleted adenovirus replicates in cancer cells although with very low efficacy. T24 cells were infected with 100 MOI of an E1-minus adenovirus expressing green fluorescent protein (Ad-GFP), and treated with 500 nM PD0332991 one day before infection and during incubation time. Under such conditions, an increase in GFP expression was observed, thus indicating E1A-independent viral replication and gene expression mediated by the activation of the adenovirus E2-early promoter.

### Example 3: Combined use of wild type adenovirus or XVir-N-31 together with different CDK4/6 inhibitors

Based upon results using the E2-early mutated adenovirus Ad-WT/E2M and Ad-GFP in combination with PD0332991, experiments were performed using different CDK4/6 inhibitors in combination with either wild type adenovirus Ad-WT or XVir-N-31. Since these agents arrest cells in phase G1, it was surprising to find that all inhibitors were able to support viral replication.

It was further examined if treatment of cells with the three clinically advanced CDK4/6 inhibitors PD-033299, LY-2835219 and LEE011 could influence the effects upon infection on cell viability, viral replication and viral titer production.

Upon treatment, all three inhibitors display similar effects on the expression and phosphorylation level of RB which has been described in numerous publications before. After an almost complete dephosphorylation and also downregulation of total protein at 24 hours, the phosphorylation level recovers partially over time. CDK2 level were upregulated upon treatment and cyclin D2 as well as cyclin E2 level were downregulated.

### Example 4: Synergistic effects arising from the combination of CDK4/6 inhibitors and oncolytic adenoviruses

CDK4/6 inhibitors PD-033299, LY-2835219 and LEE011 were combined with the infection of cells with adenovirus. Infection of cells has been done 24 hours after treatment because downstream effects on target molecules can only be detected between 8 and 24 hours after treatment.

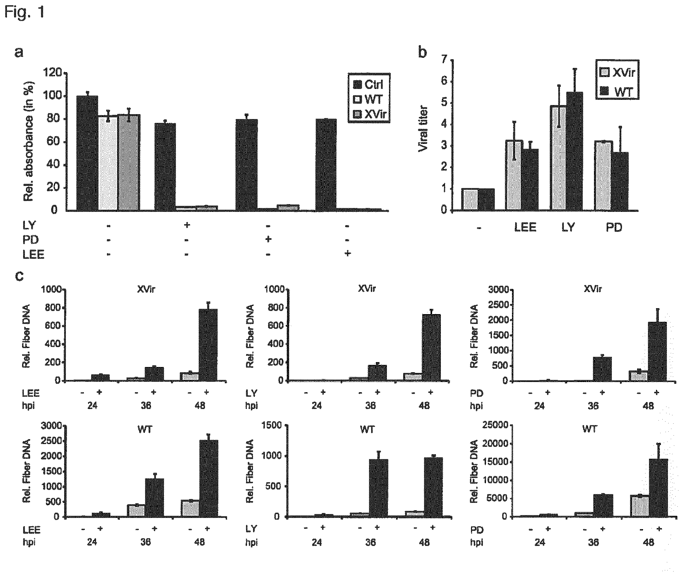

The results are shown in Fig. 1.

CDK4/6 inhibitors induced synergistic effects on cell viability, viral replication and viral titer. (a) Cells were pretreated with the three CDK4/6 inhibitors PD-033299, LY-2835219 and LEE011 for 24 hours and infected with XVir-N-31 (Moi 60) or wild type adenovirus (Moi 80). Four days past infection, cell viability was measured by an SRB assay. Graphs show averages of a minimum of three independent experiments. (b) Three days past infection, lysates were prepared from the cells and a titer test was performed on HEK293 cells. The virus titer is shown as fold change relative to control. (c) DNA was extracted from infected cells at 4, 24, 36 and 48 hpi and analysed for viral replication by using a qPCR for fiber cDNA. Values are normalized to GAPDH at 4 hpi. Graphs show representatives of at least two independent experiments. Error bars represent the standard error.

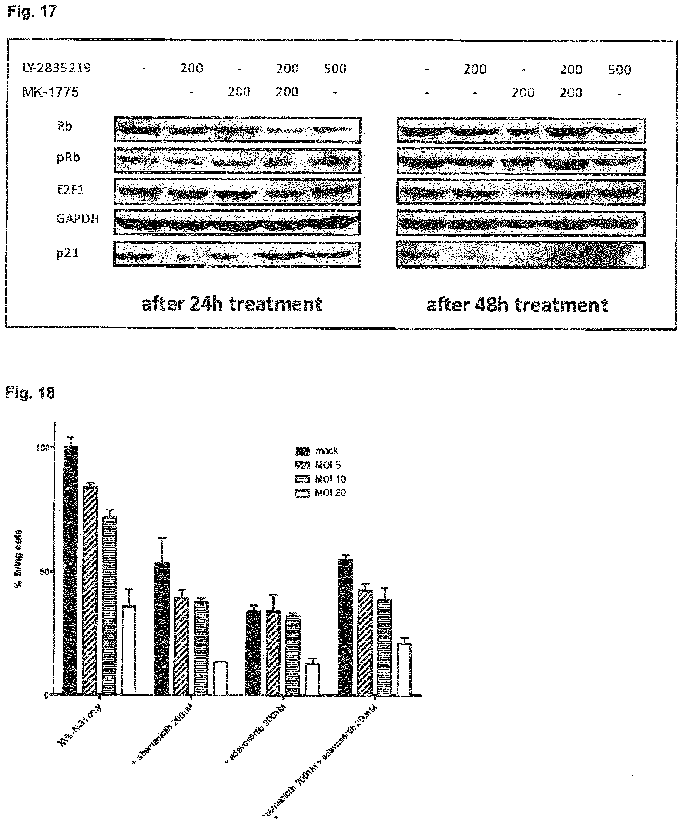

As evident from Fig. 1, all three CDK4/6 inhibitors dramatically supported cell lysis (Fig. 1a), the replication within cells (Fig 18) and the formation of viral particles (Fig 1b).

### Example 5: Effect of CDK4/6 inhibitor palbociclib (PD-033299) on the expression level of selected viral proteins

In order to analyze these effects in greater detail, expression level of selected viral proteins was determined in treated or non-treated cells. For this experiment inhibitor palbociclib (PD-033299) was used as a representative CDK4/6 inhibitor. Cells were infected with a MOI of 15. PD treatment with 500 nM took place 24 hours infection and until protein isolation took place. After 12, 24 and 36 hours protein isolation took place using 1% SDS-buffer occurred. Actin was included as a positive control. Since the loading control shows same protein levels of cellular actin in all lines, a proper comparison between the lines is ensured. hpi: hours post infection

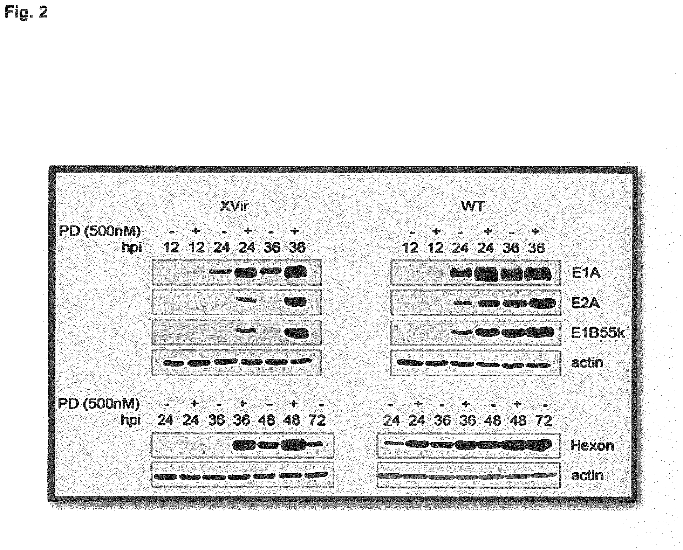

The results are indicated in Fig. 2 showing results of viral protein expression of Ad-WT and XVir-N-31 infected T24 cells in combination with the CDK4/6 inhibitor PD0332991 (PD). The viral proteins investigated in this experiment (E1A, E1B-55k, DBP (E2A) and Hexon) were all expressed at higher level in cells treated with the CDK4/6 inhibitor PD-0332991 compared to the adenovirus wild type virus. This effect could be observed as early as 12 hpi for E1A and 24 hpi for the other proteins.

### Example 6: Specificity of effects mediated by CDK4/6 inhibitors

The class of CDK4/6 inhibitors as subject to Example 5 requires expression of RB. Therefore, three RB positive and two RB negative bladder cancer derived cell lines were used and the cells treated with the combination therapy. Cell lines were pretreated for 24 hours with an IC50 concentration of PD-0332991 (T24: 500 nM, RT112: 2000 nM, 253J: 100nM) and infected with XVir-N-31(T24 MOI50, 253J MOI 25, RT112 MOI450). Values are the average of at least 2 independent experiments. Error bars show the standard error. Four dpi, cell viability was measured using SRB assays (a, c). (b, d) Lysates of cells were prepared 3 dpi and a titer test was performed on Hek293 cells. Viral titer is shown as fold change relative to control

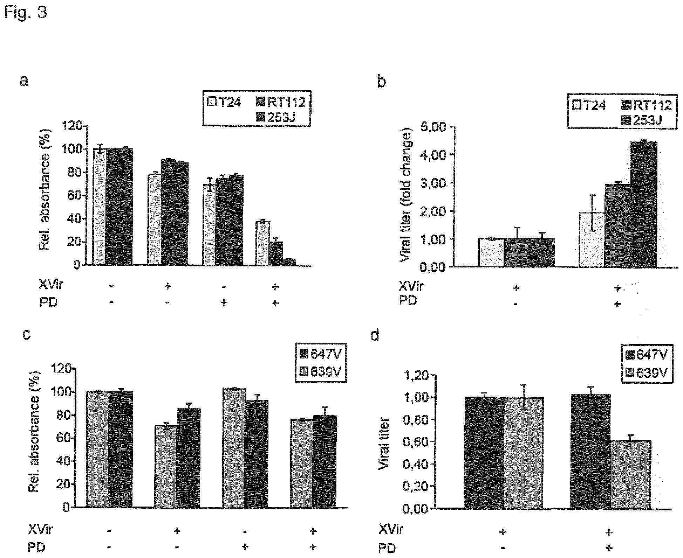

The results are shown in Fig. 3.

As evident from Fig. 3, only cell lines positive for RB showed a significant decrease in cell growth and cell viability, respectively (Fig. 3 a, c). Also, viral particle formation was only increased in RB positive cell lines upon PD-0332991 treatment (Fig. 3 b, d).

### Example 7: Effect of combination treatment of CDK4/6 inhibitor PD-0332991 with XVir-N-31

In order to investigate the effect of PD-0332991 on viral replication in the RB positive cell lines, a relative quantification of Fiber DNA copies was performed using qPCR. Bladder cancer cell lines were pretreated for 24 hours and infected with XVir-N-31 (T24 MOI 40, UMUC3 and 253J MOI 20, RT112 MOI 400). DNA was extracted 24-48 hpi and analysed for viral fiber using qPCR. Values are normalized to GAPDH. Data are representatives of at least two independent experiments; Error bars S.D.

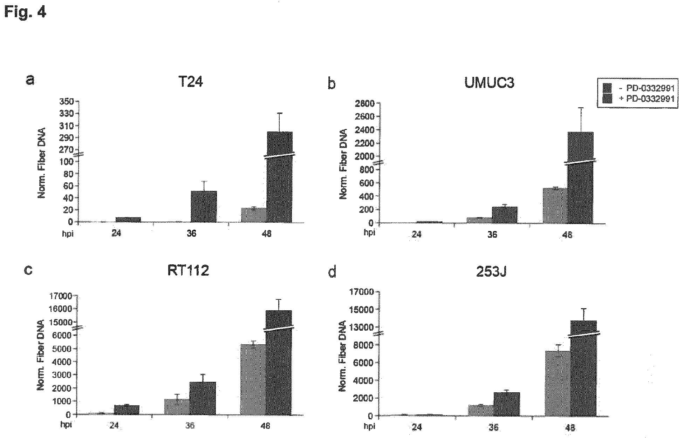

The results are shown in Fig. 4.

As may be taken form Fig. 4, combination treatment of CDK4/6 inhibitor PD-0332991 with XVir-N-31 increases viral replication dramatically.

### Example 8: Time kinetics of CDK4/6 inhibitors

Time kinetics of CDK4/6 inhibitors on the dephosphorylation and degradation of RB are around 10 hours after treatment of cells. Also, the results presented above showed partial recovery of RB downstream targets over time (Figure 1). This observation implies that time kinetics of the CDK4/6 inhibitor and the effect on viral induced cell death is an important parameter for this combination therapy as exemplified in Example 7. For application of the combination therapy, different time points for pretreatment of cells were tested. In accordance therewith, cells were treated either before (day/hour ante infection, dai/hai) or 1 hour post infection and cell growth was measured using an SRB assay. Error bars represent S.E. and the values are the average of three independent experiments

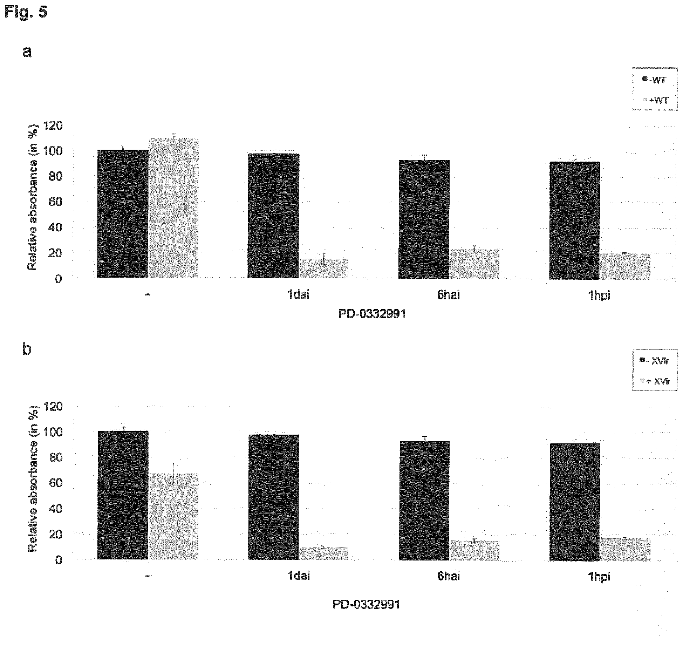

The results are shown in Fig. 5

As evident from Fig. 5, parallel treatment already was sufficient for increase in cell death.

### Example 9: Combination treatment of different adenoviruses with CDK4/6 inhibitor PD0332991

This example was performed so as to provide experimental evidence that different oncolytic adenoviruses may be used together with CDK4/6 inhibitors such as PD0332991 for cell killing, and that the observed increase in viral replication and cell killing was not restricted to XVir-N-31. In accordance therewith, T24 cancer cells with Ad-Delta24 and Onyx-015 as follows: T24 bladder cancer cells were infected with 20 MOI of the indicated oncolytic adenoviruses. Treatment with 500 nM CDK4/6 inhibitor PD0332991 took place one day before infection and for 4 days post infection. Pictures were taken 4 days post infection. The occurrence of cytopathic effect (CPE) indicates viral replication and cell killing.

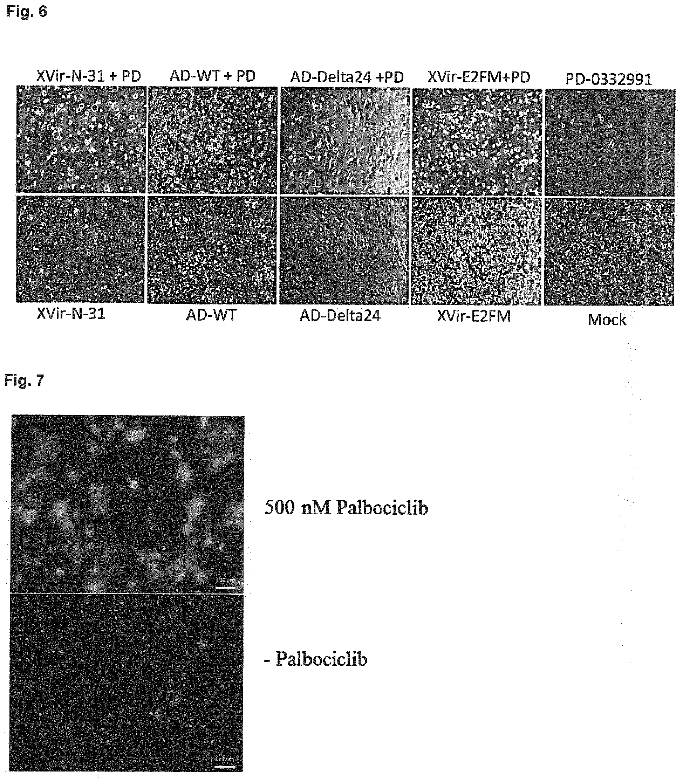

The results are shown in Fig. 6.

As may be taken from Fig. 6, CDK4/6 inhibitor PD0332991 as a representative example of CDK4/6 inhibitors reducing RB phosphorylation, increased cell killing when combined with other oncolytic adenoviruses such as Ad-Delta24 and Onyx-015.

### Example 10: Infection of T24 cells with the recombinant E1-deleted adenovirus expressing GFP (Ad-minus/GFP) in combination with Palbociclib causes increase GFP expression

100.000 T24 cells/well were seeded in 6-well plates and grown in RPMI Medium containing 10 %FCS at 5% CO₂ at 37°C. T24 cells were treated with 500 nM Palbociclib 24 hours before and again 1 hour post infection. Infection of the E1-deleted adenovirus expressing GFP (Ad-minus/GFP) took place in 400 µl Medium without serum. Pictures were taken 48 hours post infection using a fluorescence microscope with 10x magnification.

The result of fluorescence microscopic analysis of GFP expression with and without Palbociclib treatment is shown in Fig. 7.

The result shows that treatment of T24 cells with Palbociclib caused a strong increase of GFP expression which is mediated by viral DNA replication induced by Palbociclib.

### Example 11: E1A-independent viral replication in UMUC cells treated with various cell cycle inhibitors

To investigate the differences in replication of dl703 (Mantwill et al. 2013, Journal of Translational Medicine, 11, 216) under different treatment conditions, DNA-replication analysis was performed. 100.000 UMUC cells were seeded in 6-well plates and grown in DMEM medium containing 10%FCS in 5% CO₂ conditions at 37°C. 24 hours post seeding cells were treated for 24 hours with 10 µM Lee (Ribociclib), 1 µM CI-1040, 10 µM Nutlin-3a and 10 µM Roscovertine and again after infection adding an appropriate amount of inhibitors to the medium. Infection with 50 MOI dl703 (Mastadenovirus, Type C, Serotype 5 with a 3.2 kb sized deletion in E1 region) took place 24 hours post treatment. After 4 and 48 hours post infection DNA were isolated and qPCR was performed using specific primers for the viral fiber gene. Fiber fwd. 5'-AAGCTAGCCCTGCAAACATCA-3' (SEQ ID NO: 17); Fiber rev.
5'-CCCAAGCTACCAGTGGCAGTA-3' (SEQ ID NO: 18).

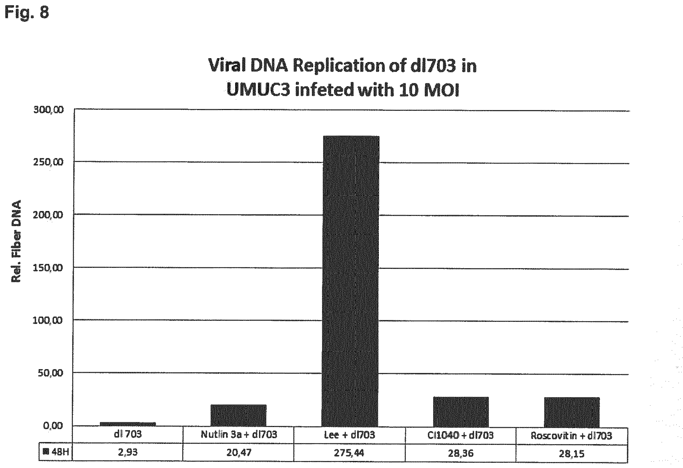

The result is shown in Fig. 8.

As evident from Fig. 8, treatment of UMUC cells with the CDK 4/6 inhibitor LEE011 (Ribociclib) caused a dramatic increase of viral DNA replication of the E1-minus adenovirus dl703 (nearly 100-fold). This increase strongly suggests that the specific induced G1-arrest by Ribociclib in conjunction with the inhibition of E2F-1 expression facilitates E1-independend adenoviral replication. In consequence, not only viruses with specific deletions in the E1A gene show enhanced adenovirus DNA replication under CDK 4/6 treatment, but even adenoviruses with complete deletion of the E1A gene show an increase in viral DNA replication.

Although the Mek-Inhibitor GI-1040 showed similar properties regarding inhibition of E2F-1 expression and G1-arrest, the replication was much lower compared to Ribociclib treated cells. This might be due to the fact that simultaneously other important cell cycle related pathways are inhibited such as MEK/ERK which is necessary for viral replication. In addition, it was shown that inhibition of the MEK/ERK-Pathway reduced particle formation more than 100-fold making it unsuitable, in a clinical setting, for combination therapy with oncolytic adenovirus replication (Schümann and Doppelstein 2016, Cancer Research, 66, 1282-1288).

### Example 12: Western Blot Analysis of UMUC cells treated with indicated cell cycles inhibitors

Western Blot analysis of UMUC cells treated with indicated concentrations of CI-1040, Roscovitine, Nutlin-3a and LEE011 (Ribociclib). 1 × 106 cells were seeded in 10 cm dishes. 24 hours post-treatment proteins were isolated using 1% SDS buffer, to achieve the disruption of the nuclear membrane. All samples were drawn up several times into a syringe to disrupt the DNA and subsequently centrifuged at 30000 rpm at 4 °C for 30 minutes. The supernatant was transferred to a new reaction tube and directly used for further steps or stored at -80 °C. To separate the proteins a sodium dodecyl sulfate polyacrylamide gel electrophoresis was performed. By means of electrophoresis for approximately two hours at 100V at 4°C, 40 µg of total proteins were loaded and probed against specific indicated antibodies.

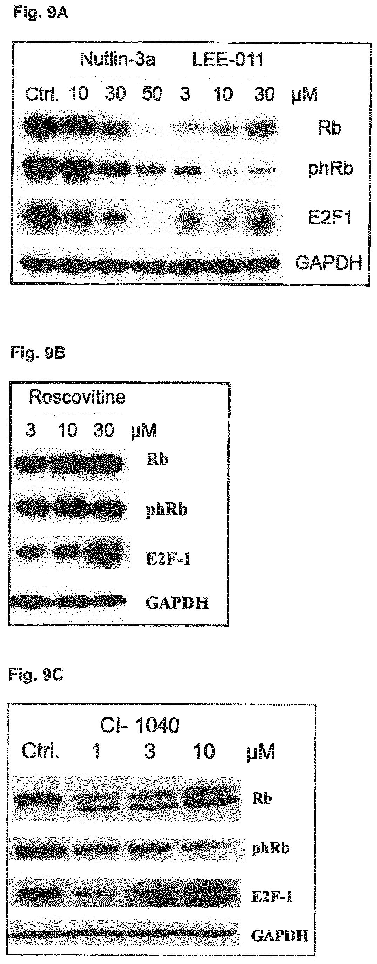

The results are shown in Figs. 9A, 9B and 9C.

As evident from Fig. 9, whereas Roscovitine and Nutlin-3a had no pronounced effect on Rb, phRB and E2F-1 expression, LEE-011 (Ribociclib) at 10 µM and MI-1040 at 1 µM induced inhibition of E2F-1 as well as Rb and phRb expression.

### Example 13: Analysis of CDK 4/6 inhibitors on viral DNA replication of the E1-deleted replication defective adenovirus dI703

For cell cycle analysis cells were seeded in 6 well plates (2,Sx10E4 c/well). 8 hours before infection with dl703, cells were treated with indicated concentration of cell cycle inhibitors. After infection with 10 MOI dl703 cells were again treated for 48 hours. Untreated cells and dl703 infected cells only, served as control. 48h post infection cells were harvest by trypsinization and fixed with 80 % ethanol while vortexing. To investigate the cell cycle status, fixed cells were centrifuged 5 min at RT and 300g and ethanol was aspirated. Cells were resuspended and washed with 1%BSA-PBS (Bovine Serum Albumin) and again centrifuged. Cells were stained with EDU and cell cycle analysis were performed using the Click-iT^{™} Plus EdU Flow Cytometry Assay Kits, Catalog nos. C10632 from Thermo Fischer. In addition, after 3x times washing with 1% BSA/PBS cells were stained with PI (Propidium Iodine,50 µg/ml). Measurement was directly performed after staining with a FACScalibur Flow Cytometry System. Data was analyzed with FlowJo software.

### Characteristics of the CDK4/6 inhibitors

CI1040: The dual specific threonine/tyrosine kinase, map kinase kinase (MEK), is a key component of the RAS/RAF/MEK/ERK signaling pathway that is frequently activated in human tumors. CI-1040 is a benzhydroxamate compound that potently inhibits MEK (Allen et al. 2003, Semin Oncol. (5 Suppl 16): 105-16) and causes G1 arrest.

Nutlin-3a: Nutlin-3, a small-molecule antagonist of MDM2, effectively restores p53 function in both normal MDM2 expression and MDM2 overexpression cell lines with wild-type p53, leading to cell cycle arrest and apoptosis (Wang et al. 2012, Acta Biochimica et Biophysica Sinica, Volume 44, Issue 8, 1 August 2012, Pages 685-691).

Roscovitine (Seliciclib or CYC202) is an experimental drug candidate in the family of pharmacological cyclin-dependent kinase (CDK) inhibitors that preferentially inhibit multiple enzyme targets including CDK2, CDK7 and CDK9, which alter the growth phase or state within the cell cycle of treated cells (Whitaker et al. 2004, Cancer Research 64, 262-272). LEE011 (Ribociclib; trade name Kisqali]) is an inhibitor of cyclin D1/CDK4 and CDK6, and is used for the treatment of certain kinds of breast cancer. The inhibition of CDK 4/6 causes G1 cell cycle arrest and inhibition of E2F-1 expression (Kim S. et al., Oncotarget. 2018 Oct 16;9(81):35226-35240; Yang C et al., Oncogene (2017)36,2255-2264).

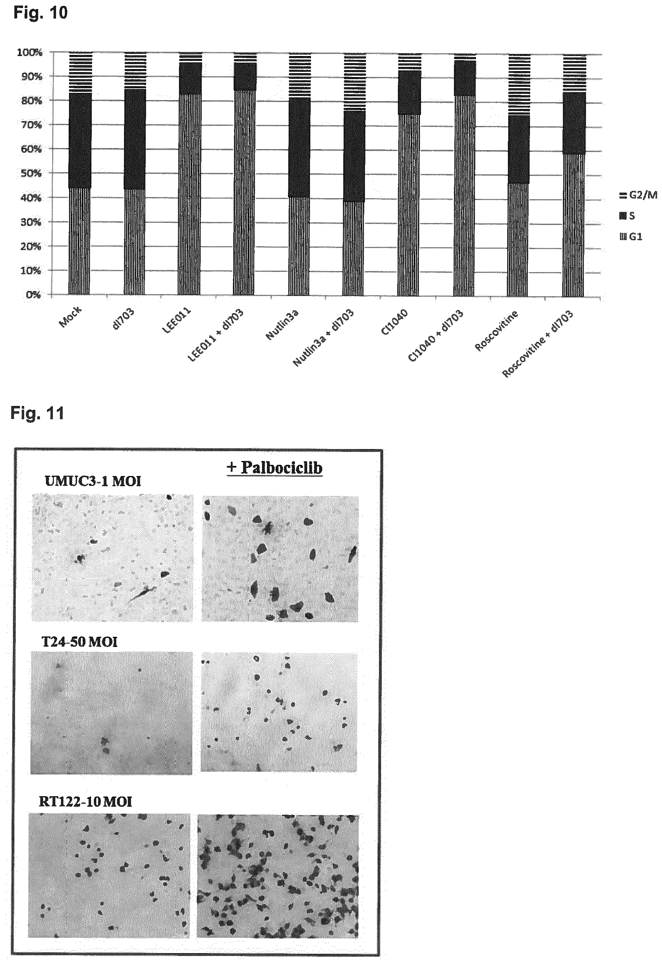

The results are shown in Fig. 10.

The CDK 4/6 inhibitors LEE011 (Ribociclib) and CI-1040 induced a clear G1-arrest. Treatment with Roscovitine showed a slight increase of G2/m arrested cells. Nutlin-3a had only little or no effect on the cell cycle in the used concentration. Infection of UMUC cells with the recombinant E1-deleted (having no E1A protein) adenovirus dl703 did not change the cell cycle distribution significantly.

### Example 14: Palbocilib increased adenovirus hexon staining in vitro post treatment

Bladder cell lines RT112, T24 and UMUC were seeded in 6-well plates (2 x105 cells/well). One day post seeding cells were treated with 500 nM Palbociclib for 24 hours before and again 1 hour post infection. Infection with indicated MOIs of AD-WT took place in 400 µl DMEM-Medium without serum. Hexon staining was performed according to manufacturer instructions using Adeasy Viral Titer Kit from Agilent (cat: 972500) two days post infection.

The result is shown in Fig. 11.

As evident from Fig. 11, treatment of Palbociclib (500 nM) as an exemplary CDK4/6 inhibitor increased hexon positive cells significantly in Palbociclib treated cells 48 hours post infection as indicated by the brown/red colour. The conclusion must be reached, that more cells under Palbociclib treatment are capable to produce viral particles and show increase viral DNA replication, since adenovirus hexon expression occurs exclusive onset of viral replication.

From the results subject to Examples 10 to 14, it is evident that only CDK4/6 inhibitors but no other cell cycle inhibitors are capable of increasing replication and gene expression of replication defective adenovirus (dl703 lacking the E1 genes) and Ad-GFP. Furthermore, a CDK4/6 inhibitor in order to provide such increased viral replication and gene expression must cause G1 arrest of (infected) cells and inhibition of F2F1 expression.

### Example 15: Treatment of T24 cells using triple therapy comprising XVir-N-31, Palbociclib and a PARP inhibitor

In order to show the efficacy of a triple therapy of T24 cells using triple therapy comprising XVir-N-31, Palbociclib and a PARP inhibitor (BMN673 (Talazolarib)), a potency assay was carried out.

12.500 T24 cells were seeded per well in 12-well plates and grown over-night in RPMI Medium containing 10 % FCS at 37°C. Inhibitor-treatment of cells occurred 24 h past cell seeding and again 1 hour after infection by adding indicated concentration to the medium. Infection of cells took place 24 h past inhibitor-treatment in 250 µl medium without serum. Fixation and SRB-staining took place at 4 days post infection. PD, Palbociclib; PARPi: BMN673.

For SRB staining, the medium was removed by aspiration. Cells were fixed with 1 ml (per well) 10 % cold TCA at 4°C for 1 hour. TCA was removed by aspiration and cell layers were washed 4 × with tap water. Cells were stained with 1 ml (per well) 0.5% SRB (sulforhodamine B) in 1% acetic acid for 30 min. Unbound SRB was removed in five washing steps with 1 ml 1% acetic acid /well; after each washing step, acetic acid was removed by aspiration. Plates were air-dried for 2 hrs. To solubilize the SRB stained cells, 200 µl of 10 mM Tris base was added to each well. Afterwards 20 µl, respectively, was dispensed into wells of a 96 well plate. The 96 well plate was loaded into an Elisa-plate reader and absorption of the samples was measured at 560 nm. Mock treated cells were set 100 % cell survival.

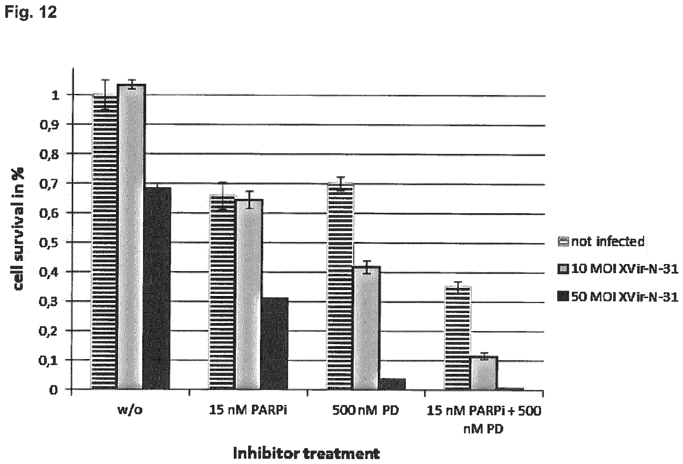

The result is shown in Fig. 12.

The result shown in figure 12 clearly demonstrates that the triple therapy consisting of Palbociclib, BMN673 and XVir-N-31 exhibited a superior performance against mono- or combination therapy regarding cell killing. Nearly 90% cell killing could be achieved using 10 MOI of XVir-N-31 in combination with PARP inhibitor PARPi (BMN673) and CDK4/6 inhibitor Palbociclib (PD). The combination of PARPi and Palbociclib without XVir-N-31 killed only 65% of the cells. T24 cells and UMUC cells are sensitive to CDK 4/6-Inhibitors (providing G1 arrest with E2F-1 down-regulation).

### Example 16: Kinetics of triple therapy comprising XVir-N-31, Palbociclib and a PARP inhibitor

In order to show the kinetics of a triple therapy of T24 cells using triple therapy comprising XVir-N-31, Palbociclib and a PARP inhibitor (BMN673 (Talazolarib)), a potency assay was carried out and the potency assessed at different points in time.

3000 T24 cells were seeded per well in 12-well plates and grown over-night in RPMI Medium containing 10 %FCS at 37°C. Inhibitor-treatment of cells occurred 24 h past cell seeding and again 1 hour after infection by adding indicated concentration to the medium. Infection of cells took place 24 h past inhibitor-treatment in 250 µl Medium without serum. Fixation and SRB-staining took place at 1-5 days post infection (dpi: days post infection). 15 nM PARPi correspond to the IC-80 value in T24 cells.

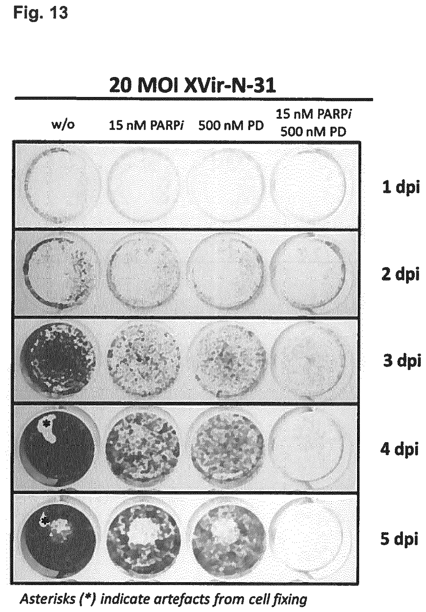

The results are shown Fig. 13.

As evident from Fig. 13, triple therapy using apart from XVir-N-31 a CDK4/6 inhibitor (Palbociclib (PD) and a PARP inhibitor PARPI (BMN673) is, also from a kinetic point of view, much more effective than a monotherapy using XVir-N-31 only or a combination therapy using XVir-N-31 an either the PARP inhibitor or the CDK4/6 inhibitor. Importantly, the re-growth of tumor cells was significantly reduced at day 4 and 5 in the CDK 4/6 sensitive cell lines UMUC and T24 (dpi: days post infection).

### Example 17: Kinetics of triple therapy comprising XVir-N-31, Palbociclib and a PARP inhibitor

In order to show the kinetics of a triple therapy of UMUC cells using triple therapy comprising XVir-N-31, Palbociclib and a PARP inhibitor (BMN673 (Talazolarib)), a potency assay was carried out and the potency assessed at different points in time.

Seeding of UMUC-3: 3000 cells were seeded per well in 12-well plates and grown over-night in DMEM Medium containing 10 %FCS at 37°C. Inhibitor-treatment of cell occurred 24 h past seeding and again 1 hour after infection by adding indicated concentration to the medium. Infection of cells took place 24 h past inhibitor-treatment. Fixation and SRB-staining took place at 1-6 days post infection (dpi: days post infection). 160 nM PARPi correspond to the IC-80 value in UMUC3 cells.

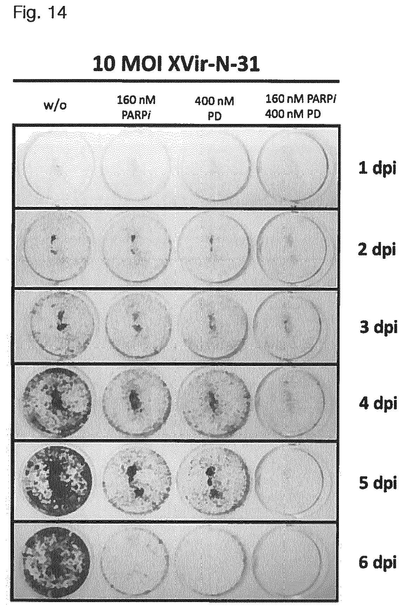

The result is shown in Fig. 14.

The results shown in Fig. 14 clearly demonstrate that the triple therapy consisting of Palbociclib, BMN673 and XVir-N-31 exhibited superior performance against mono- or combination therapy. Importantly, the re-growth of tumor cells was significantly reduced at day 4 and 5 in the CDK 4/6 sensitive cell lines UMUC and T24 (dpi: days post infection).

### Example 18: Triple therapy comprising XVir-N-31, a CDK4/6 inhibitor and a bromodomain inhibitor

5000 T24 cells were seeded in 12 well plates and grown in 1 ml RPMI-Medium containing 10 % FCS. Next day cells were treated with 500 nM Palbociclib and 300 nM JQ-1. 24 hours post treatment cells were infected with indicated MOIs of XVir-N-31 in 200 µl RPMI-Medium containing no FCS. After 1 hour 800 µl RPMI-Medium containing 10 % FCS were added into each well. In addition, 500 nM Palbociclib and 300 nM JQ-1 were added to the medium. SRB-Staining took place 5 days post infection. Mock treated cells were set 100 % cell survival.

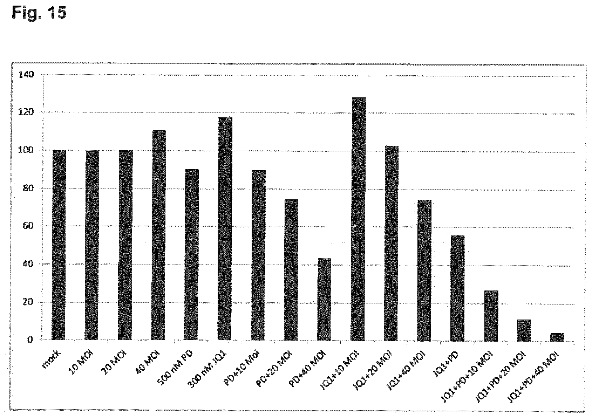

The results are shown in Fig. 15.

As evident from Fig. 15, the bromodomain inhibitor JQ1 increased the cell killing capacity of XVir-N-31 in combination with the CDK 4/6 inhibitor Palbociclib at low MOIs. Light-microscopic analysis 48 hours post infection reveals already massive cell death in JQ1/Palbociclib/XVir-N-31 treated cells. The conclusion must be reached that JQ-1 increased viral transcription and thereby viral replication in Palbociclib treated cells, since monotherapy with 300 nM JQ1 alone did not increase cell killing of XVir-N-31 at 10 and 20 MOI.

A prerequisite for the observed enhancement of JQ-1 in adenovirus infected cancer cells is the ability of Palbociclib to induce G1-arrest. In cells which are resistant against Palbociclib (see Example 18, identical treatment procedure), no increase of cell killing was observed. This observation was in sharp contrast to Baojie Lv et al. 2018, Scientific reports, 8, 11554, where cells were treated with concentrations of JQ1, causing no G1-arrest and no Palbociclib was used in conjunction.

### Example 19: Triple therapy comprising XVir-N-31, a CDK4/6 inhibitor and a bromodomain inhibitor

100.000 SK-N-MC cells/well were seeded in 12-well plates and grown in RPMI Medium containing 10 %FCS at 5% CO2 at 37°C. Cells were treated with 200 nM Abemaciclib + 500 nM JQ1 24 hours before and again 1 hour post infection by adding appropriate amount to the medium. Infection of XVir-N-31 took place in 500 µl in RPMI Medium without serum. SRB-Staining took place 5 days post infection. Mock treated cells were set 100 % cell survival.

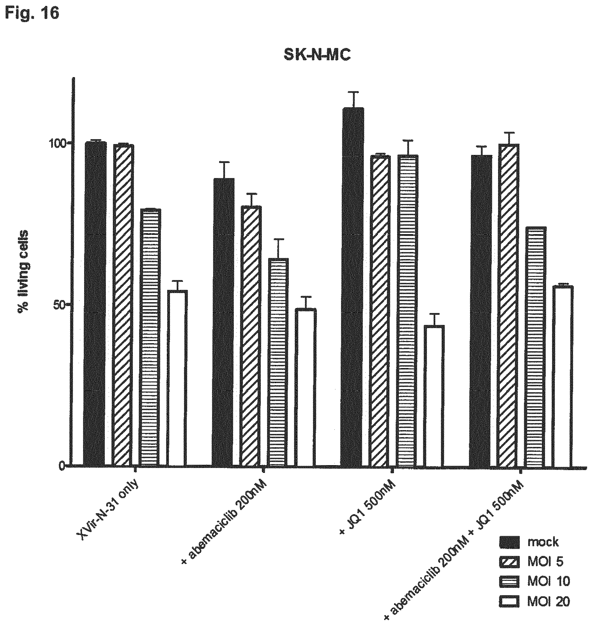

The results are shown in Fig. 16.

It is established that SK-N-MC cells are resistant against CDK 4/6 Inhibitors which thus does not cause a G1-arest. The addition of JQ1 did not increase cell killing of CDK 4/6 (Abemaciclib) resistant SK-N-MC cells, indicating that the CDK 4/6 mediated G1-arrest is a prerequisite of the JQ1mediated effect on cell killing.

Thus, Figs. 16 (as well as Fig. 15) show that bromodomain inhibitors targeting BRD2, BRD3, BRD4 increase the cell killing effect of XVir-N-3 even further under the premise that CDK 4/6 Inhibitors induces a G1-arrest in treated cells.

### Example 20: Western Blot Analysis of SK-N-MC cells treated with CDK 4/6 Inhibitor LY-2835219 (Abemaciclib) and the Wee-Inhibitor MK-1775 (Adavosertib)

1 × 10⁶ cells were seeded in 10 cm dishes. 24 hours post-treatment proteins were isolated using 1% SDS buffer, to achieve the disruption of the nuclear membrane. All samples were drawn up several times into a syringe to disrupt the DNA and subsequently centrifuged at 30000 rpm at 4 °C for 30 minutes. The supernatant was transferred to a new reaction tube and directly used for further steps or stored at -80 °C. To separate the proteins a sodium dodecyl sulfate polyacrylamide gel electrophoresis was performed. By means of electrophoresis for approximately two hours at 100V at 4°C, 40 µg of total proteins were loaded and probed against specific indicated antibodies.

The result is shown in Fig. 17.

It is known that SK-N-MC cells are resistant to Abenaciclib treatment (Dowless M et al., 2018, Clin Cancer Res: 24, 6028-6039). Wee1 is a critical component of the G2/M cell cycle checkpoint control and mediates cell cycle arrest by regulating the phosphorylation of CDC2. Inhibition of Wee1 by MK1775 has been reported to enhance the cytotoxic effect of DNA damaging agents in different types of carcinomas. Several studies have demonstrated that pharmacological inhibition of Wee1 by the small molecule kinase inhibitor MK-1775 leads to removal of CDC2 phosphorylation at Tyr15 in tumor cells (Kreahling et al. 2013, PLoS One. 8(3), e 57523). Although a strong G1-arrest is observed in the combination treatment no change in Rb and E2F-1 expression is observed.

### Example 21: Triple therapy comprising XVir-N-31, a CDK4/6 inhibitor Abemaciclib and Adavosertib (Wee-inhibitor MK-1775)

100.000 SK-N-MC cells/well were seeded in 12-well plates and grown in RPMI Medium containing 10 %FCS at 5% CO₂ at 37°C. Cells were treated with 200 nM Abemaciclib 24 hours before and again 1 hour post infection by adding appropriate amount to the medium. Infection of XVir-N-31 took place in 500 µl in RPMI Medium without serum. SRB-Staining took place 5 days post infection. Mock treated cells were set 100 % cell survival.

The results are shown in Fig. 18.

Figs. 17 (and 18) demonstrate that the combination of the CDK 4/6 Inhibitor Abemaciclib and the Wee-Inhibitor MK-1775 induced G1 arrest without inhibition of E2F-1. The potency assay in Fig. 18 shows that this combination did not enhance the cell killing effect of the oncolytic adenovirus XVir-N-31. These results clearly demonstrate that the induced G1-arrest by the combination of the CDK 4/6 Inhibitor Abemaciclib and the Wee-Inhibitor MK-1775 did not facilitate XVir-N-31 cell killing capacity. Thus, the inhibition of E2F-1 expression is a further requirement to enhance viral oncolysis.

### Example 22: G1 arrest in combination with E2F-1 inhibition is a prerequisite for enhanced cell killing of XVir-N-31 in combination with CDK 4/6 inhibitors

48 hours post treatment cells were washed twice took place with PBS (containing RNase A, 100 U/ml). Cells were trypsinized and centrifuged at 1500 rpm, 4°C for 5 min. Cells are fixed by adding slowly 1 ml of ice-cold 80% Ethanol drop by drop to the pellet and incubated overnight. Staining was performed by adding 1ml of staining solution (Propidium Iodine,50 µg/ml) to the cells and incubating 30-60 min at RT with gentle rocking. MK: MK-1775; LY: LY-2835219.

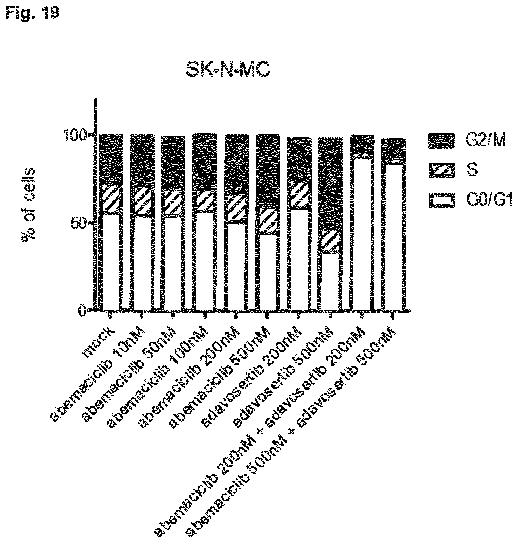

The result is shown in Fig. 19.

As evident from Fig. 19, treatment of SK-M-NC cells with LY (Abemaciclib) had no effect of the cell cycle. MK-1775 treatment alone caused at 500 nM an increase of cells in G2/M. Combination of both caused a strong G1-arrest.

### Example 23: Role of E2F-1 expression on viral DNA replication

I. 2 × 10⁵ T24, A549, and HeLa cells were seeded in per well in a 6 well plate and grown in , 1.5 ml RPMI 1640 Medium containing (or DMEM-Medium) 10 % FBS, penicillin/streptomycin and non-essential amino acids. The following day, 30 pmol siRNA - whether negative control siRNA (Qiagen #1022076) or siE2F1 (Sigma # NM_005225, siRNA ID SASI_Hs01_00162220) was diluted in 150 µL Opti-MEM Medium and 9 µl Lipofectamine RNAiMAX was prepared in 150 µL Opti-MEM. The siRNA-solution and the Lipofectamine RNAiMAX solution were mixed and incubated for 5 minutes. The mixture was dropwise added to the cells. After 48 hours RNA was isolated and RT-qPCR was performed.

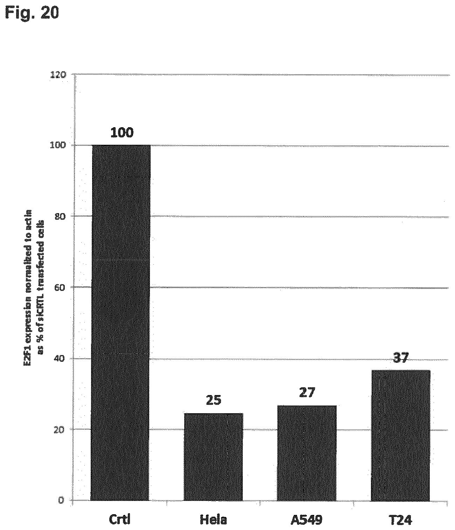

The result is shown in Fig. 20. As evident from Fig. 20, E2-early expression is decreased

II. For each well of a 6 well plate, 2 × 10⁵ T24 cells were seeded in 1.5 ml RPMI 1640 Medium containing 10 % FBS, penicillin/streptomycin and non-essential amino acids. The following day, 30 pmol siRNA - whether negative control siRNA (Qiagen #1022076) or siE2F1 (Sigma # NM_005225, siRNA ID SASI_Hs01_00162220) was diluted in 150 µL Opti-MEM Medium and 9 µl Lipofectamine RNAiMAX was prepared in 150 µL Opti-MEM. The siRNA-solution and the Lipofectamine RNAiMAX solution were mixed and incubated for 5 minutes. The mixture was dropwise added to the T24 cells. Infection took place 48h later during incubating the cells with 10 MOI of ADWTRGD in 400 µl of serum free medium and rocking the plate every 10-15 minutes. After 1 h, 1.6 ml full medium was added. RNA isolation was done 24 h after infection.

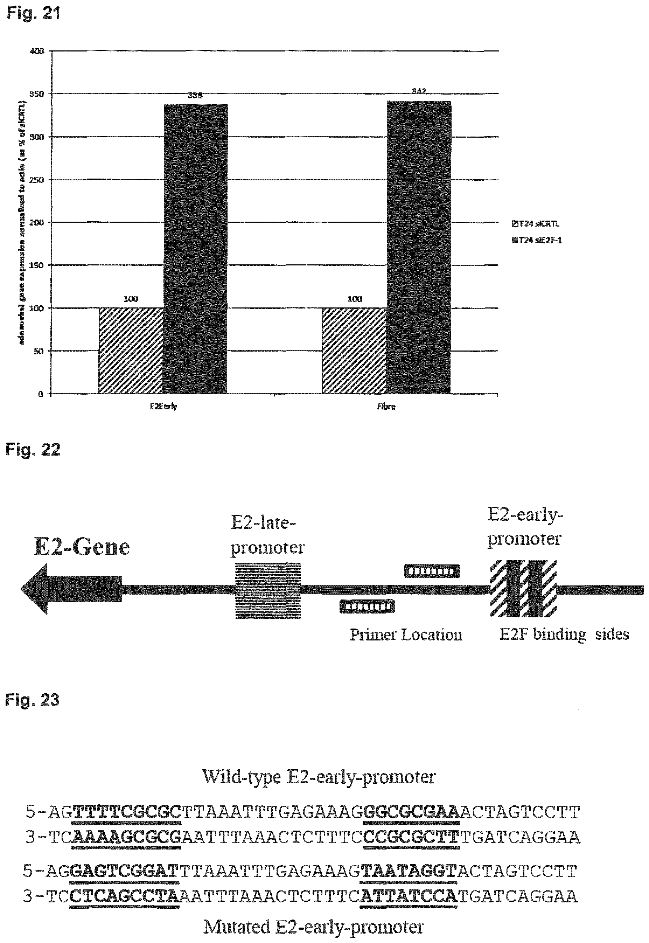

The result is shown in Fig. 21.

III. Cells were rinsed with cold PBS and disrupted by adding 500 µl lysibuffer from the MirVana Kit , Thermo Fisher catalonumber AM1560, the lysates were collected with a spatula, and pipetted into a 1.5 ml tube. For the organic extraction, 50 µl Homogenate Additive was added and Samples were incubated on ice for 10 min. 500 µl of acid-phenol: chloroform was added and samples were vortexed for 60s and incubated on ice for 2 minutes. Samples were centrifuged at 14 000 x g, at room temperature for 5 min to separate the aqueous and organic phases. The upper phase was carefully transferred to a new tube and an add equal amount of Isopropanol was added. After incubation at room temperature for 10 min, RNA was precipitated (14 000 x g, 4 °C, 30 min.) and washed twice with 1 mL of 75% ethanol (centrifuge 7500 × g, 4°C, 5 min.). RNA was air dried for 5-10 minutes and resuspended in 20-50µl RNase-free water and resolved by shaking at 500rpm, 55°C for 10min.and measured by Nanodrop. After DNA digestion (Deoxyribonuclease I, Invitrogen Cat.No. 18068-015) using 1 µg RNA Sample mit 1 µl 10 x DNAse I reaction buffer, nuclease-free water to 9 µl volume, 1 µl DNaseI (1 U/µl) , incubation 15 min at room temperature, Inactivating the DNAseI by the addition of 1 µl 25 mM EDTA solution, heating for 10 min at 65 °C. Reverse transcription was performed using the High-Capacity cDNA Reverse Transcription Kit (Thermo Fisher/ Applied Biosystems^{™} Catalog number: 4368814 ).Using random hexamere for the transcription for fibre and actin PCR, and using E2 Early Primer for the transcription for the E2Early-PCR.

### Used primers and siRNAs

E2 Earlyfw: CCGTCATCTCTACAGCCCAT (SEQ ID NO: 19)
E2 Earlyrev: GGGCTTTGTCAGAGTCTTGC (SEQ ID NO: 20)
fiberfw: AAGCTAGCCCTGCAAACATCA (SEQ ID NO: 21)
fiberrev: CCCAAGCTACCAGTGGCAGTA (SEQ ID NO: 22)
Actinfw: TCACCCACACTGTGCCCATCTACG (SEQ ID NO: 23)
Actinrev: CAGCGGAACCGCTCATTGCCAATGG (SEQ ID NO: 24)
E2F1 fw: CATCCCAGGAGGTCACTTCTG (SEQ ID NO: 25)
E2F1 rev: GACAACAGCGGTTCTTGCTC(SEQ ID NO: 26)

### Contro siRNA

Sense UUCUCCGAACGUGUCACGUdTdT (SEQ ID NO: 27)
Antisense: ACGUGACACGUUCGGAGAAdTdT (SEQ ID NO: 28)

### E2F-1 siRNA

CUGAGGAGUUCAUCAGCCU[dT][dT] (SEQ ID NO: 29)
AGGCUGAUGAACUCCUCAG[dT][dT] (SEQ ID NO: 30)

To proof the role of E2-early expression by RT-qPCR it is absolute necessary to choose the right primer. The primer location should be between the E2-early and the E2-late promoter. Otherwise the E2-late promoter will strongly influence the results. The location of the primers is shown in Fig. 22.

As evident from Figs. 20 and 21, down-regulation of E2F1 by siRNA causes increase in E2-early expression. This could only be explained by the repressive role of E2F1 in E2-early expression. If E2F-1 would be an activator, a decrease of E2-early expression would be the consequence. In addition, siRNA against E2F-1 mimic the effect of CDK 4/6 inhibitors, which also inhibits E2F-1 expression (Yang C et al., Oncogene 2017, 36,2255-2264).

### Example 24: Recombinant adenovirus with mutations of the two E2F-binding sites in the adenovirus E2-early promoter shows increased E2-early expression.

A mutant adenovirus was generated having mutations at the two E2F-binding sites of the adenoviral E2 early promoter. The promoter of both the wild type E2 early promoter and the mutant E2 early promoter is shown in Fig. 23.

RNA-Expression analysis was carried out in AdWT-RGD and AdE2Fm (contain also the RGD motive) infected T24 cells obtained by RT-qPCR at 24 hours post infection. AD-WT gene expression was set to 100%. The method was identical to the one described in section III of Example 23.

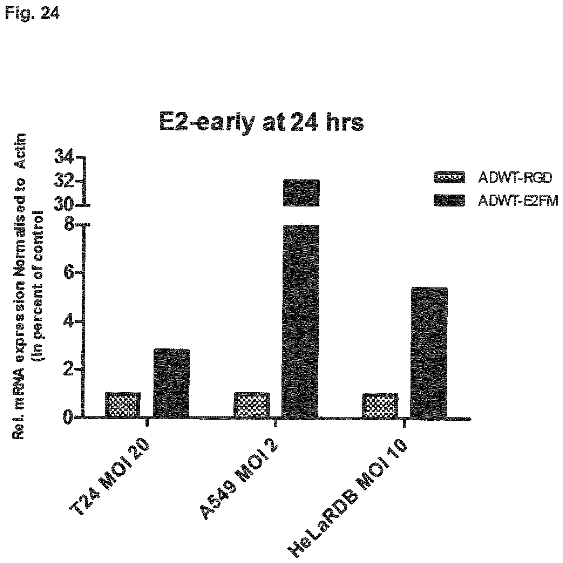

The result is shown in Fig. 24.

As evident from Fig. 24, expression of E2-early gene expression was higher in AdE2Fm infected cells compared to AD-WT infected cells. Therefore, it must be concluded that E2F-1 is playing a repressive role in E2-early promoter activation. This is in sharp contrast to current understanding, where E2F-1 is postulated to be an activator (DeCaprio JA, Virology. 2009 Feb 20;384(2):274-84.

It is well known, that the structure of the E2-region in all currently known oncolytic adenoviruses is build up as shown in Fig. 22. Thus, the mode of action of E2F-1 is identical as described here. In consequence, all of them, i.e. all oncolytic adenoviruses can be used in combination with CDK 4/6 inhibitors, including ColoAd1 and Delta-24-RGD.

ColoAd1 can be characterized as follows:
Enadenotucirev (formerly ColoAd1) is a tumor-selective chimeric adenovirus with demonstrated preclinical activity. The capsid of ColoAd1 is from Ad11p, a serotype with limited seroprevalence in humans. EnAd infects cells by binding to CD46 and/or desmoglein 2,6 both widely expressed on many carcinoma cells. Most of the EnAd genome is derived from Ad11p with a large deletion in E3 and a smaller deletion in E4. In addition, the E2B region consists of a chimera of sequences from Ad11p and Ad3. The E4 deletion in EnAd is in E4ORF4, which in Ad5 encodes a protein that inactivates protein phosphatase2A and thereby activates protein translation machinery as well as regulating activity of E1A protein in a feedback inhibitory loop. These deletions, perhaps combined with the chimeric E2B region, probably contribute to the striking cancer-selective replication of EnAd (Deyer et al., Mol Ther Oncolytics. 2017, 16; 5: 62-74)

Delta-24-RGD (DNX-2401) can be characterized as follows:
Delta-24-RGD (DNX-2401) is a conditional replication-competent oncolytic virus engineered to preferentially replicate in and lyse tumor cells with abnormality of p16/RB/E2F pathway. Fueyo et al., Oncogene. 2000 Jan 6;19(1):2-12. A mutant oncolytic adenovirus targeting the Rb pathway produces anti-glioma effect in vivo; Dai B. et al., Mol Cancer Therapy. 2017 Apr;16(4):662-670.

## Claims

1. A combination comprising an oncolytic adenovirus and a CDK4/6 inhibitor, wherein the adenovirus is replicating in a YB-1 dependent manner and wherein the adenovirus is lacking expression of E1A13S.

2. The combination of claim 1, wherein the adenovirus is replication deficient in cells which lack YB-1 in the nucleus, but is replicating in cells which have YB-1 in the nucleus.

3. The combination of any ones of claims 1 to 2, wherein the combination further comprises a PARP inhibitor.

4. The combination of any one of claims 1 to 3, wherein the combination further comprises a bromodomain inhibitor.

5. The combination of any one of claims 1 to 4 for use in a method for the treatment of a tumor or cancer.

6. An adenovirus for use in a method for the treatment of a tumor or cancer in a subject, wherein the method comprises administering to the subject an adenovirus and a CDK4/6 inhibitor, wherein the adenovirus is defined as in any one of claims 1 to 2.

7. A CDK4/6 inhibitor for use in a method for the treatment of a tumor or cancer in a subject, wherein the method comprises administering to the subject an adenovirus and the CDK4/6 inhibitor, wherein the adenovirus is defined as in any one of claims 1 to 2.

8. The combination of any one of claims 1 to 4, the combination for use of claim 5, the adenovirus for use of claim 6, and the CDK4/6 inhibitor for use of claim 7, wherein the adenovirus is selected from the group comprising XVir-N-31, dl520, AdΔ24, AdΔ24-RGD, dl922-947, E1Ad/01/07, dl1119/1131, CB 016, VCN-01, E1Adl1107, E1Adl1101, ORCA-010, Enadenotucirev and viruses lacking an expressed viral oncogene which is capable of binding a functional Rb tumor suppressor gene product.

9. The combination of any one of claims 1 to 4 and 8, the combination for use of any one of claims 5 and 8, the adenovirus for use of any one of claims 6 and 8, and the CDK4/6 inhibitor for use of any one of claims 7 and 8, wherein the adenovirus is XVir-N-31.

10. The combination of any one of claims 1 to 4 and 8 to 9, the combination for use of any one of claims 5 and 8 to 9, the adenovirus for use of any one of claims 6 and 8 to 9, and the CDK4/6 inhibitor for use of any one of claims 7 and 8 to 9, wherein the CDK4/6 inhibitor is a CDK4/6 inhibitor arresting cells in the G1 phase and inhibiting E2F1.

11. The combination of any one of claims 1 to 4 and 8 to 10, the combination for use of any one of claims 5 and 8 to 10 the adenovirus for use of any one of claims 6 and 8 to 10, and the CDK4/6 inhibitor for use of any one of claims 7 and 8 to 10, wherein the CDK4/6 inhibitor is selected from the group comprising palbociclib which is also referred to as PD 0332991, abemaciclib which is also referred to as LY-2835219, ribociclib which is also referred to as LEE011, Trilaciclib which is also referred to as G1T28, and Dinaciclib.

12. The combination for use of any one of claims 5 and 8 to 11, the adenovirus for use of any one of claims 6 and 8 to 11, and the CDK4/6 inhibitor for use of any one of claims 7 and 8 to 11, wherein the disease tumor or cancer is expressing Rb or is Rb-positive.

13. The combination for use of any one of claims 5 and 8 to 12, the adenovirus for use of any one of claims 6 and 8 to 12, and the CDK4/6 inhibitor for use of any one of claims 7 and 8 to 12, wherein the cells of the tumor cells have a resistance to or are insensitive to one or several pharmaceutically active agents and/or radiation.

14. The combination for use of any one of claims 5 and 8 to 13, the adenovirus for use of any one of claims 6 and 8 to 13, and the CDK4/6 inhibitor for use of any one of claims 7 and 8 to 13, wherein the tumor or cancer contains YB-1 in the cell nucleus independent of the cell cycle.

15. The combination for use of any one of claims 5 and 8 to 14, the adenovirus for use of any one of claims 6 and 8 to 14, and the CDK4/6 inhibitor for use of any one of claims 7 and 8 to 14, wherein the disease is selected from the group comprising bladder cancer, breast cancer, metastatic breast cancer (mBC), melanoma, glioma, pancreatic cancer, hepatocellular carcinoma, lung adenocarcinoma, sarcoma, ovarian cancer, renal cancer, prostate cancer, and leukemia.

## Patentansprüche

1. Kombination umfassend einen onkolytischen Adenovirus und einen CDK4/6-Inhibitor, wobei der Adenovirus YB-1 abhängig repliziert und wobei dem Adenovirus die Expression von E1A13S fehlt.

2. Kombination nach Anspruch 1, wobei der Adenovirus replikationsdefizient ist in Zellen, denen YB-1 im Nukleus fehlt, aber in Zellen repliziert, die YB-1 im Nukleus aufweisen.

3. Kombination nach einem der Ansprüche 1 bis 2, wobei die Kombination weiter einen PARP-Inhibitor umfasst.

4. Kombination nach einem der Ansprüche 1 bis 3, wobei die Kombination weiter einen Bromdomänen-Inhibitor umfasst

5. Kombination nach einem der Ansprüche 1 bis 4, zur Verwendung in einem Verfahren für die Behandlung eines Tumors oder einer Krebserkrankung.

6. Adenovirus zur Verwendung in einem Verfahren für die Behandlung eines Tumors oder einer Krebserkrankung in einem Lebewesen, wobei das Verfahren umfasst Verabreichen eines Adenovirus und eines CDK4/6-Inhibitors an das Lebewesen, wobei der Adenovirus wie in einem der Ansprüche 1 bis 2 definiert ist.

7. CDK4/6-Inhibitor zur Verwendung in einem Verfahren für die Behandlung eines Tumors oder einer Krebserkrankung in einem Lebewesen, wobei das Verfahren umfasst Verabreichen eines Adenovirus und des CDK4/6-Inhibitors an das Lebewesen, wobei der Adenovirus wie in einem der Ansprüche 1 bis 2 definiert ist.

8. Kombination nach einem der Ansprüche 1 bis 4, Kombination zur Verwendung nach Anspruch 5, Adenovirus zur Verwendung nach Anspruch 6 und CDK4/6-Inhibitor zur Verwendung nach Anspruch 7, wobei der Adenovirus ausgewählt ist aus der Gruppe umfassend XVir-N-31, dl520, AdΔ24, AdΔ24-RGD, dl922-947, E1Ad/01/07, dl1119/1131, CB 016, VCN-01, E1Adl1107, E1Adl1101, ORCA-010, Enadenotucirev und Viren, denen ein exprimiertes virales Onkogen fehlt, das in der Lage ist, ein funktionelles Rb-Tumorsuppressor-Genprodukt zu binden.

9. Kombination nach einem der Ansprüche 1 bis 4 und 8, Kombination zur Verwendung nach einem der Ansprüche 5 und 8, Adenovirus zur Verwendung nach einem der Ansprüche 6 und 8 und CDK4/6-Inhibitor zur Verwendung nach einem der Ansprüche 7 und 8, wobei der Adenovirus XVir-N-31 ist.

10. Kombination nach einem der Ansprüche 1 bis 4 und 8 bis 9, Kombination zur Verwendung nach einem der Ansprüche 5 und 8 bis 9, Adenovirus zur Verwendung nach einem der Ansprüche 6 und 8 bis 9 und CDK4/6-Inhibitor zur Verwendung nach einem der Ansprüche 7 und 8 bis 9, wobei der CDK4/6-Inhibitor ein CDK4/6-Inhibitor ist, der Zellen in der G1-Phase arretiert und E2F1 inhibiert.

11. Kombination nach einem der Ansprüche 1 bis 4 und 8 bis 10, Kombination zur Verwendung nach einem der Ansprüche 5 und 8 bis 10, Adenovirus zur Verwendung nach einem der Ansprüche 6 und 8 bis 10 und CDK4/6-Inhibitor zur Verwendung nach einem der Ansprüche 7 und 8 bis 10, wobei der CDK4/6-Inhibitor ausgewählt ist aus der Gruppe umfassend Palbociclib, das auch als PD 0332991 bezeichnet wird, Abemaciclib, das auch als LY-2835219 bezeichnet wird, Ribociclib, das auch als LEE011 bezeichnet wird, Trilaciclib, das auch als G1T28 bezeichnet wird, und Dinaciclib.

12. Kombination zur Verwendung nach einem der Ansprüche 5 und 8 bis 11, Adenovirus zur Verwendung nach einem der Ansprüche 6 und 8 bis 11 und CDK4/6-Inhibitor zur Verwendung nach einem der Ansprüche 7 und 8 bis 11, wobei die Erkrankung, der Tumor oder die Krebserkrankung Rb exprimiert oder Rb-positiv ist.

13. Kombination zur Verwendung nach einem der Ansprüche 5 und 8 bis 12, Adenovirus zur Verwendung nach einem der Ansprüche 6 und 8 bis 12 und CDK4/6-Inhibitor zur Verwendung nach einem der Ansprüche 7 und 8 bis 12, wobei die Zellen der Tumorzellen eine Resistenz aufweisen oder gegenüber einem oder mehreren pharmazeutisch aktiven Agenzien und/oder Strahlung unempfindlich sind.

14. Kombination zur Verwendung nach einem der Ansprüche 5 und 8 bis 13, Adenovirus zur Verwendung nach einem der Ansprüche 6 und 8 bis 13 und CDK4/6-Inhibitor zur Verwendung nach einem der Ansprüche 7 und 8 bis 13, wobei der Tumor oder die Krebserkrankung YB-1 im Zellkern unabhängig vom Zellzyklus enthält.

15. Kombination zur Verwendung nach einem der Ansprüche 5 und 8 bis 14, Adenovirus zur Verwendung nach einem der Ansprüche 6 und 8 bis 14 und CDK4/6-Inhibitor zur Verwendung nach einem der Ansprüche 7 und 8 bis 14, wobei die Erkrankung ausgewählt ist aus der Gruppe umfassend Blasenkarzinom, Brustkrebs, metastatischen Brustkrebs (mBC), Melanom, Gliom, Pankreaskarzinom, hepatozelluläres Karzinom, Lungenadenokarzinom, Sarkom, Ovarialkarzinom, Nierenkarzinom, Prostatakrebs und Leukämie.

## Revendications

1. Combinaison comprenant un adénovirus oncolytique et un inhibiteur de CDK4/6, dans laquelle l'adénovirus se réplique d'une manière dépendante de YB-1 et dans laquelle l'adénovirus est dépourvu d'expression de E1A13S.

2. Combinaison selon la revendication 1, dans laquelle l'adénovirus est déficient en réplication dans les cellules qui manquent de YB-1 dans le noyau, mais se réplique dans les cellules qui ont YB-1 dans le noyau.

3. Combinaison selon l'une quelconque des revendications 1 à 2, dans laquelle la combinaison comprend en outre un inhibiteur de PARP.

4. Combinaison selon l'une quelconque des revendications 1 à 3, dans laquelle la combinaison comprend en outre un inhibiteur de bromodomaine.

5. Combinaison selon l'une quelconque des revendications 1 à 4 pour son utilisation dans une méthode de traitement d'une tumeur ou d'un cancer.

6. Adénovirus pour son utilisation dans une méthode de traitement d'une tumeur ou d'un cancer chez un sujet, dans lequel la méthode comprend l'administration au sujet d'un adénovirus et d'un inhibiteur de CDK4/6, dans lequel l'adénovirus est défini selon l'une quelconque des revendications 1 à 2.

7. Inhibiteur de CDK4/6 pour son utilisation dans une méthode de traitement d'une tumeur ou d'un cancer chez un sujet, dans lequel la méthode comprend l'administration au sujet d'un adénovirus et de l'inhibiteur de CDK4/6, dans lequel l'adénovirus est défini comme selon l'une quelconque des revendications 1 à 2.

8. Combinaison selon l'une quelconque des revendications 1 à 4, combinaison pour son utilisation selon la revendication 5, adénovirus pour son utilisation selon la revendication 6 et inhibiteur de CDK4/6 pour son utilisation selon la revendication 7, dans lesquels l'adénovirus est choisi dans le groupe comprenant XVir-N-31, dl520, AdΔ24, AdΔ24-RGD, dl922-947, E1Ad/01/07, dl1119/1131, CB 016, VCN-01, E1Adl1107, E1Adl1101, ORCA-010, Enadenotucirev et les virus dépourvus d'un oncogène viral exprimé qui est capable de se lier à un produit fonctionnel du gène suppresseur de tumeur Rb.

9. Combinaison selon l'une quelconque des revendications 1 à 4 et 8, combinaison pour son utilisation selon l'une quelconque des revendications 5 et 8, adénovirus pour son utilisation selon l'une quelconque des revendications 6 et 8, et inhibiteur de CDK4/6 pour son utilisation selon l'une quelconque des revendications 7 et 8, dans lesquels l'adénovirus est XVir-N-31.

10. Combinaison selon l'une quelconque des revendications 1 à 4 et 8 à 9, combinaison pour son utilisation selon l'une quelconque des revendications 5 et 8 à 9, adénovirus pour son utilisation selon l'une quelconque des revendications 6 et 8 à 9, et inhibiteur de CDK4/6 pour son utilisation selon l'une quelconque des revendications 7 et 8 à 9, dans lesquels l'inhibiteur de CDK4/6 est un inhibiteur de CDK4/6 arrêtant les cellules en phase G1 et inhibant E2F1.

11. Combinaison selon l'une quelconque des revendications 1 à 4 et 8 à 10, combinaison pour son utilisation selon l'une quelconque des revendications 5 et 8 à 10, adénovirus pour son utilisation selon l'une quelconque des revendications 6 et 8 à 10, et inhbiteur de CDK4/6 pour son utilisation selon l'une quelconque des revendications 7 et 8 à 10, dans lesquels l'inhibiteur de CDK4/6 est choisi dans le groupe comprenant le palbociclib qui est également appelé PD 0332991, l'abemaciclib qui est également appelé LY-2835219, le ribociclib qui est également appelé LEE011, le trilaciclib qui également appelé G1T28 et le Dinaciclib.

12. Combinaison pour son utilisation selon l'une quelconque des revendications 5 et 8 à 11, adénovirus pour son utilisation selon l'une quelconque des revendications 6 et 8 à 11, et inhibiteur de CDK4/6 pour son utilisation selon l'une quelconque des revendications 7 et 8 à 11, dans lesquels la tumeur pathologique ou le cancer exprime Rb ou est Rb-positif.

13. Combinaison pour son utilisation selon l'une quelconque des revendications 5 et 8 à 12, adénovirus pour son utilisation selon l'une quelconque des revendications 6 et 8 à 12, et inhibiteur de CDK4/6 pour son utilisation selon l'une quelconque des revendications 7 et 8 à 12, dans lesquels les cellules des cellules tumorales ont une résistance ou sont insensibles à un ou plusieurs agents pharmaceutiquement actifs et/ou au rayonnement.

14. Combinaison pour son utilisation selon l'une quelconque des revendications 5 et 8 à 13, adénovirus pour son utilisation selon l'une quelconque des revendications 6 et 8 à 13, et inhibiteur de CDK4/6 pour son utilisation selon l'une quelconque des revendications 7 et 8 à 13, dans lesquels la tumeur ou le cancer contient YB-1 dans le noyau de la cellule indépendamment du cycle de la cellule.

15. Combinaison pour son utilisation selon l'une quelconque des revendications 5 et 8 à 14, adénovirus pour son utilisation selon l'une quelconque des revendications 6 et 8 à 14, et inhibiteur de CDK4/6 pour son utilisation selon l'une quelconque des revendications 7 et 8 à 14, dans lesquels la maladie est choisie dans le groupe comprenant le cancer de la vessie, le cancer du sein, le cancer du sein métastatique (mBC), le mélanome, le gliome, le cancer du pancréas, le carcinome hépatocellulaire, l'adénocarcinome pulmonaire, le sarcome, le cancer de l'ovaire, le cancer du rein, le cancer de la prostate et la leucémie.
